# EUROPEAN PATENT APPLICATION

(11) **EP 4 671 233 A1**
(43) Date of publication of application: **31.12.2025**
(21) Application number: 24832541.7
(22) Date of filing: 01.07.2024
(51) Int. Cl.: C07C 237/22, C07C 231/12

(54) **NOVEL HEMIASTERLIN DERIVATIVE AND USE THEREOF**

(30) Priority: 29.06.2023 KR 20230084458
(71) Applicant: Pinotbio, Inc., Gyeonggi-do 16506 (KR)
(72) Inventor: JUNG, Doo Young, Suwon-si, Gyeonggi-do 16506 (KR); MATHI, Gangadhar Rao, Suwon-si, Gyeonggi-do 16506 (KR)
(74) Representative: Prüfer & Partner mbB Patentanwälte · Rechtsanwälte
(86) International application number: PCT/KR2024/009233
(87) International publication number: WO 2025/005765

(57) **Abstract**

The present invention provides a novel hemiasterlin derivative, a preparation method therefor, and a use thereof. In the present invention, an anticancer effect is confirmed using a novel hemiasterlin derivative with improved stability.

## Description

### Technical Field

The present invention provides novel hemiasterlin derivatives, a method for preparing thereof, and uses thereof. The novel hemiasterlin derivatives synthesized according to the present invention exhibit significant inhibitory effects on the viability of cancer cell lines, and therefore can be advantageously utilized as a safe form of anticancer agent.

### Related Art

Hemiasterlin can be isolated from marine sponges such as Cymbastela sp., Hemiasterella minor, Siphonochalina sp., and Auletta sp. (Talpir et al., Tetrahedron Letters, vol. 35, no. 25, pp. 4453-4456, 1994).

Natural hemiasterlin is known as an agent that inhibits the formation of microtubules, which are essential for cancer cell division, and has been actively investigated for the treatment of cancers such as ovarian cancer. However, issues concerning safety still remain.

Accordingly, the present inventors have identified the structure of hemiasterlin derivatives with improved safety as therapeutic agents and have confirmed anticancer activity comparable to that of natural hemiasterlin, thereby accomplishing the present invention.

### SUMMARY OF THE INVENTION

### TECHNICAL PROBLEM

The present invention, in order to solve the aforementioned problems, identifies a novel hemiasterlin derivative structure with improved safety, and intends to provide the same as a drug for preventing or treating cancer, in forms such as a drug-linker or drug-carrier conjugate (ADC).

### TECHNICAL SOLUTION

In order to achieve the above object, the present invention provides a compound of Chemical Formula 1, an isomer thereof, a pharmaceutically acceptable salt thereof or a solvate thereof as a novel derivative of hemiasterlin.

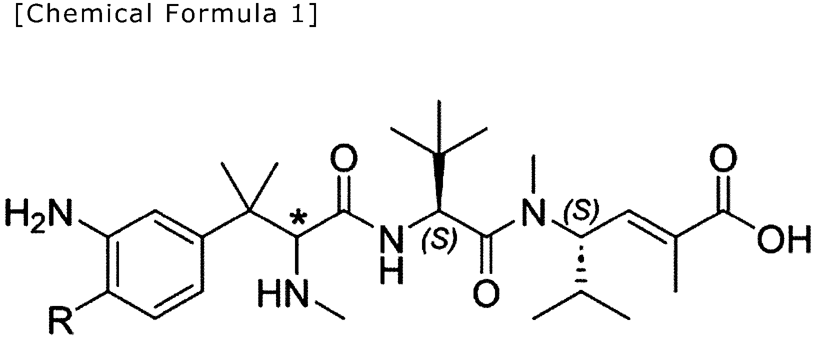

Wherein R, in Chemical Formula 1, is selected from the group consisting of halogen, hydroxy, carboxyl group, C1-C6 alkyl and C1-C6 alkoxy.

In the present invention, a derivative or a stereoisomer of the compound of Chemical Formula 1 includes compounds represented by following Chemical Formula 2 to 7, but is not limited thereto.

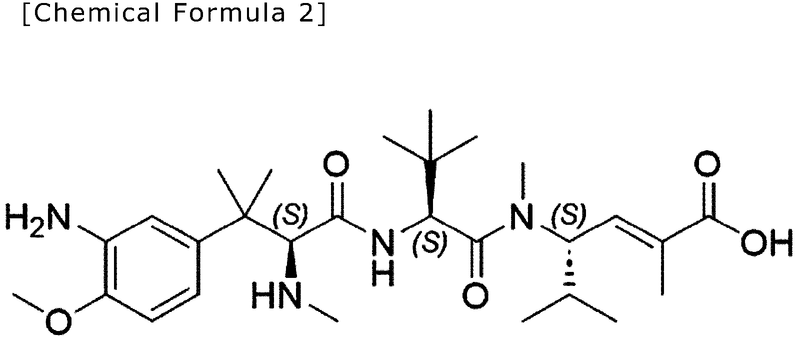

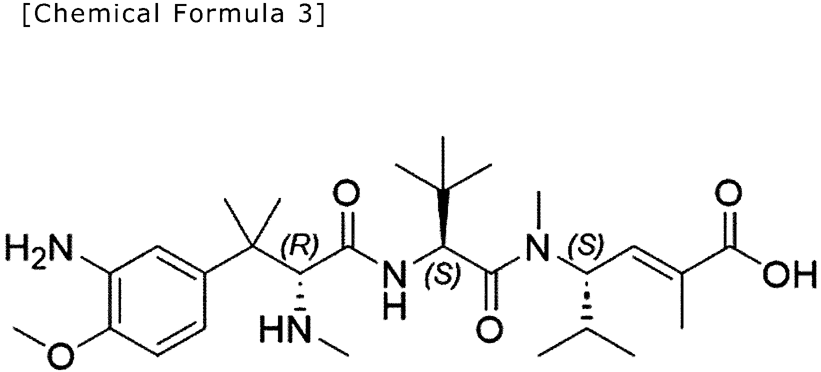

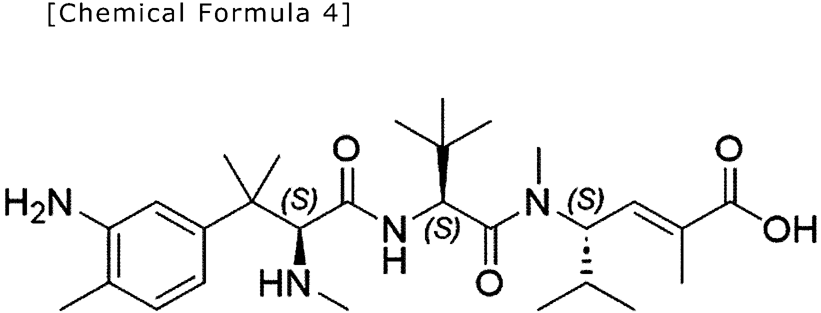

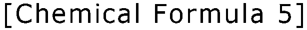

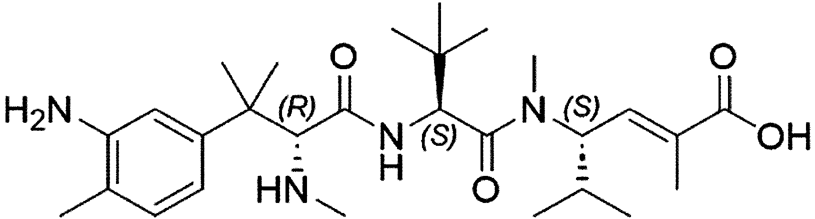

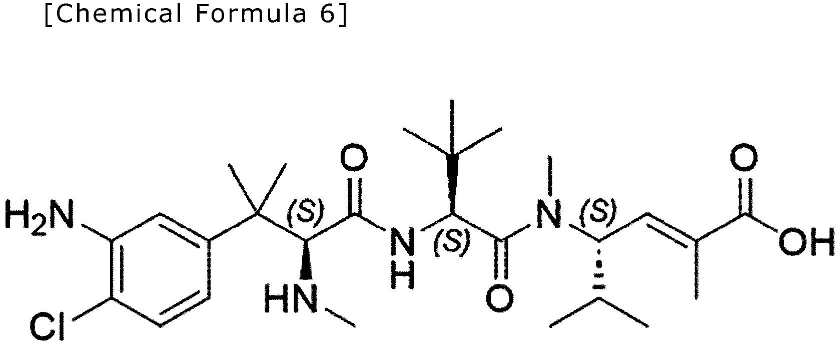

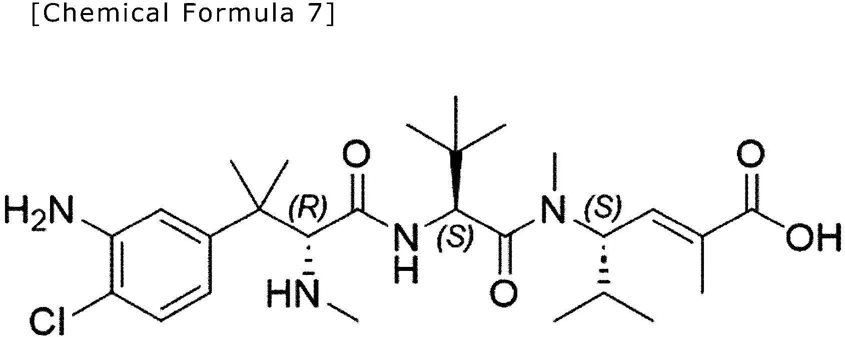

The present invention also provides a "carrier-drug conjugate" or a "carrier-linker-drug conjugate," comprising a compound of Chemical Formula 1, a derivative thereof, an isomer thereof, a pharmaceutically acceptable salt thereof, or a solvate thereof; and a carrier conjugated to the compound.

In another embodiment of the present invention, there is provided a "drug-linker" comprising a compound of Chemical Formula 1, a derivative thereof, an isomer thereof, a pharmaceutically acceptable salt thereof, or a solvate thereof; and a linker conjugated to the compound.

In the present invention, the linker may comprise, but is not limited to, GGFG or val-cit, and any linker conventionally used in the art may be employed without limitation.

In the present invention, "carrier" means a substance capable of selectively and specifically delivering the compound according to the invention to a target site, for example, to cancer cells, and may be an antibody, peptide, repebody, and/or aptamer, but is not limited thereto, and is preferably an antibody. For example, the carrier of the present invention may be an antibody, peptide, repebody, or aptamer that specifically binds to one or more substances (antigens) selected from the group consisting of 4-1BB, 5T4, integrin, Activin, amyloid beta, angiopoietin (angiopoietin 1 or 2), angiopoietin-like protein 3, B-cell maturation antigen (BCMA), B-cell activating factor (BAFF), B7-H3, complement 5, CCR4, CCR5, CCL11, CD2, CD3, CD4, CD6, CD11a, CD16A, CD19, CD20, CD22, CD25, CD27, CD28, CD30, CD32B, CD33, CD38, CD40, CD45, CD46, CD47, CD52, CD56, CD62, CD70, CD73, CD74, CD79b, CD80, CD105, CD123, CD154, CD166, CD262, CD278, CD319, CD326, carcinoembryonic antigen (CEA), CGRP, Claudin-18, c-Met, CSF-1, CSF-1 receptor, CTLA4, DLL3, EGF receptor, hemophilia factor, Fc receptor, FGF23, folate receptor, GD2, glucocorticoid-induced TNF receptor (GITR), Glypican 3, GM-CSF, HER2, HER3, TROP2, hepatocyte growth factor (HGF), interferon receptor, interferon gamma, IgE, IGF-1 receptor, interleukin-1, interleukin-2 receptor, interleukin-4, interleukin-4 receptor, interleukin-5, interleukin-5 receptor, interleukin-6, interleukin-6 receptor, interleukin-8, interleukin-12/23, interleukin-13, interleukin-17A, interleukin-17 receptor A, interleukin-23, interleukin-31 receptor, interleukin-36 receptor, lymphocyte-activation gene 3 (LAG3), lysyl oxidase homolog 2 (LOXL2), mesothelin, mucin-1, mucin-16, Nectin-4, nerve growth factor (NGF), OX40, proprotein convertase subtilisin/kexin type 9 (PCSK9), PD-1, PD-L1, phospholipase C, RANKL (receptor activator of nuclear factor kappa-B ligand), tyrosine-protein kinase transmembrane receptor (ROR1), sialic acid-binding Ig-like lectin 15 (Siglec-15), transforming growth factor beta (TGF-β), TIGIT (T-cell immunoreceptor with immunoglobulin and ITIM domain), T-cell immunoglobulin and mucin-domain containing-3 (Tim-3), tissue factor, tissue factor pathway inhibitor (TFPI), TORP-2, tumor necrosis factor (TNF), thymic stromal lymphopoietin (TSLB), colony stimulating factor 1 receptor (CSF1R), vascular endothelial growth factor (VEGF), VEGF receptor, and von Willebrand Factor (vWF).

For example, in the present invention, the antibody may include Urelumab, Utomilumab, Bebtelovimab, Aducanumab, Bapinezumab, Crenezumab, Donanemab, Gantenerumab, Lecanemab, Solanezumab, Nesvacumab, Evinacumab, Enoblituzumab, Omburtamab, Belimumab, Ianalumab, Tabalumab, Bertilimumab, Mogamulizumab, Leronlimab, Siplizumab, Foralumab, Muromonab-CD3, Otelixizumab, Teplizumab, Ibalizumab, Tregalizumab, Zanolimumab, Itolizumab, Efalizumab, Inebilizumab, Tafasitamab, Tositumomab, Ocrelizumab, Ofatumumab, Rituximab, Ublituximab, Veltuzumab, Epratuzumab, Basiliximab, Daclizumab, Varlilumab, Lulizumab, Iratumumab, Lintuzumab, Daratumumab, Felzartamab, Isatuximab, Mezagitamab, Bleselumab, Dacetuzumab, Iscalimab, Lucatumumab, Mitazalimab, Sotigalimab, Dapirolizumab, Apamistamab, Ligufalimab, Magrolimab, Alemtuzumab, Crizanlizumab, Inclacumab, Cusatuzumab, Oleclumab, Milatuzumab, Galiximab, Carotuximab, Adecatumumab, Eptinezumab, Erenumab, Fremanezumab, Galcanezumab, Zolbetuximab, Onartuzumab, Eculizumab, Pozelimab, Ravulizumab, Lacnotuzumab, Axatilimab, Cabiralizumab, Emactuzumab, Ipilimumab, Quavonlimab, Tremelimumab, Zalifrelimab, Cetuximab, Depatuxizumab, Futuximab, Imgatuzumab, Matuzumab, Modotuximab, Necitumumab, Nimotuzumab, Panitumumab, Tomuzotuximab, Zalutumumab, Batoclimab, Nipocalimab, Rozanolixizumab, Burosumab, Farletuzumab, Dinutuximab, Naxitamab, Ragifilimab, Gimsilumab, Lenzilumab, Mavrilimumab, Namilumab, Otilimab, Plonmarlimab, Codrituzumab, Margetuximab, Pertuzumab, Trastuzumab, Datopotamab, Patritumab, Seribantumab, Duligotuzumab, Ficlatuzumab, Rilotumumab, Alomfilimab, Anifrolumab, Emapalumab, Ligelizumab, Omalizumab, Cixutumumab, Dalotuzumab, Figitumumab, Ganitumab, Teprotumumab, Bermekimab, Canakinumab, Gevokizumab, Briakinumab, Ustekinumab, Anrukinzumab, Cendakimab, Lebrikizumab, Tralokinumab, Brodalumab, Bimekizumab, Ixekizumab, Secukinumab, Brazikumab, Guselkumab, Mirikizumab, Risankizumab, Tildrakizumab, Nemolizumab, Imsidolimab, Spesolimab, Pascolizumab, Dupilumab, Depemokimab, Mepolizumab, Reslizumab, Benralizumab, Clazakizumab, Olokizumab, Siltuximab, Sirukumab, Ziltivekimab, Levilimab, Sarilumab, Satralizumab, Tocilizumab, Abituzumab, Favezelimab, Fianlimab, Ieramilimab, Relatlimab, Simtuzumab, Abagovomab, Oregovomab, Tanezumab, Ivuxolimab, Rocatinlimab, Tavolimab, Telazorlimab, Vonlerolizumab, Alirocumab, Bococizumab, Ebronucimab, Evolocumab, Frovocimab, Ongericimab, Tafolecimab, Dostarlimab, Balstilimab, Camrelizumab, Cemiplimab, Geptanolimab, Nivolumab, Pembrolizumab, Penpulimab, Pidilizumab, Prolgolimab, Retifanlimab, Sasanlimab, Serplulimab, Sintilimab, Spartalizumab, Tislelizumab, Toripalimab, Ezabenlimab, Zimberelimab, Atezolizumab, Avelumab, Cosibelimab, Sugemalimab, Durvalumab, Envafolimab, Suvratoxumab, Denosumab, Zilovertamab, Elotuzumab, Domvanalimab, Etigilimab, Ociperlimab, Tiragolumab, Vibostolimab, Surzebiclimab, Cobolimab, Sabatolimab, Concizumab, Marstacimab, Adalimumab, Golimumab, Infliximab, Certolizumab, Conatumumab, Tigatuzumab, Tezepelumab, Gatipotuzumab, Cabiralizumab, Bevacizumab, Brolucizumab, Ranibizumab, Olinvacimab, Icrucumab, Ramucirumab, Caplacizumab, Abrilumab, Etrolizumab, Vedolizumab, Intetumumab, Natalizumab, Obrindatamab, Elranatamab, Linvoseltamab, Teclistamab, Epcoritamab, Glofitamab, Mosunetuzumab, Odronextamab, Flotetuzumab, Vibecotamab, Catumaxomab, Cibisatamab, Talquetamab, Ubamatamab, Emfizatamab, Blinatumomab, Amivantamab, Emicizumab, Zenocutuzumab, Zanidatamab, Tibulizumab, Naptumomab, Belantamab, Pivekimab, Praluzatamab, Coltuximab, Denintuzumab, Loncastuximab, Ibritumomab, Inotuzumab, Epratuzumab, Moxetumomab, Brentuximab, Gemtuzumab, Vadastuximab, Lorvotuzumab, Polatuzumab, Tusamitamab, Telisotuzumab, Rovalpituzumab, Depatuxizumab, Farletuzumab, Mirvetuximab, Disitamab, Anetumab, Enfortumab, Sacituzumab, Vobarilizumab, Cadonilimab, Vudalimab, Tebotelimab, Ivonescimab, Erfonrilimab, Ozoralizumab, Faricimab, Vanucizumab, and Navicixizumab, but is not limited thereto.

The present invention also provides "a pharmaceutical composition for preventing or treating cancer" comprising a compound of Chemical Formula 1, an isomer thereof, a pharmaceutically acceptable salt thereof, or a solvate thereof, and provides the "use for preventing or treating cancer" of a compound represented by Chemical Formula 1.

Wherein, the compound of Chemical Formula 1 may be provided in a form conjugated to a carrier via a linker.

In the present invention, the cancer includes, pseudomyxoma, intrahepatic cholangiocarcinoma, hepatoblastoma, liver cancer, thyroid cancer, colon cancer, testicular cancer, myelodysplastic syndrome, glioblastoma, oral cancer, lip cancer, mycosis fungiodes, acute myeloid leukemia, acute lymphoblastic leukemia, basal cell carcinoma, ovarian epithelial carcinoma, ovarian germ cell carcinoma, male breast cancer, brain cancer, pituitary adenoma, multiple myeloma, gallbladder cancer, bile duct cancer, colorectal cancer, chronic myelogenous leukemia, chronic lymphocytic leukemia, retinoblastoma, choroidal melanoma, ampulla of Vater cancer, bladder cancer, peritoneal cancer, parathyroid cancer, adrenal cancer, paranasal sinus cancer, non-small cell lung cancer, tongue cancer, astrocytoma, small cell lung cancer, pediatric brain cancer, pediatric lymphoma, childhood leukemia, small bowel cancer, meningioma, esophageal cancer, glioma, renal pelvis cancer, kidney cancer, heart cancer, duodenal cancer, malignant soft tissue cancer, malignant bone cancer, malignant lymphoma, malignant mesothelioma, malignant melanoma, eye cancer, vulvar cancer, ureteral cancer, urethral cancer, unknown primary neoplasm, gastric lymphoma, gastric cancer, gastric carcinoma, gastrointestinal stromal cancer, Wilms' cancer, breast cancer, Triple-negative breast cancer(TNBC), sarcoma, penile cancer, pharyngeal cancer, gestational trophoblastic disease, cervical cancer, endometrial cancer, uterine sarcoma, prostate cancer, metastatic bone cancer, metastatic brain cancer, mediastinal cancer, rectal cancer, occipital carcinoid tumors, vaginal cancer, spinal cancer, vestibular schwannoma, pancreatic cancer, salivary gland cancer, Kaposi's sarcoma, Paget's disease, tonsil cancer, squamous cell carcinoma, lung adenocarcinoma, lung cancer, squamous cell lung cancer, skin cancer, anal cancer, rhabdomyosarcoma, laryngeal cancer, pleural cancer, blood cancer, and thymic carcinoma, but is not limited thereto.

### ADVANTAGEOUS EFFECTS

The present invention provides novel hemiasterlin derivatives and a method for preparing thereof. The novel hemiasterlin derivatives according to the present invention can be utilized as safe and effective pharmaceutical compositions.

### BRIEF DESCRIPTION OF THE DRAWINGS

FIG. 1 illustrates the results of measuring cell viability according to treatment concentration after incubating lung cancer cell line (A549) for 3 days following treatment with the compound of Chemical Formula 2, SC209, and MMAE.
FIG. 2 illustrates the results of measuring cell viability according to treatment concentration after incubating head and neck squamous cell carcinoma cell line (FaDu) for 3 days following treatment with the compound of Chemical Formula 2, SC209, and MMAE.

### BEST MODE FOR CARRYING OUT THE INVENTION

Hereinafter, various Examples are provided to aid in the understanding of the present invention. It should be understood that the following Examples are provided merely for illustrative purposes to facilitate the understanding of the invention, and are not intended to limit the scope of protection of the invention to these Examples.

The present invention provides, as derivatives of hemiasterlin, a compound of Chemical Formula 1, an isomer thereof, a pharmaceutically acceptable salt thereof, or a solvate thereof.

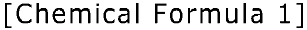

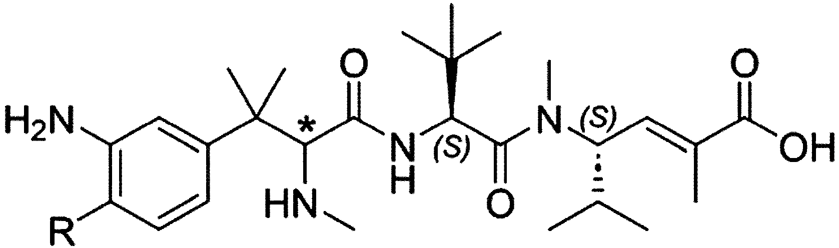

In Chemical Formula 1, R is selected from the group consisting of halogen, hydroxy, carboxyl, C1-C6 alkyl, and C1-C6 alkoxy.

In the present specification, hemiasterlin refers to a compound having the molecular formula C₃₀H₄₆N₄O₄(molecular weight: 526.7 g/mol) and having the following chemical structure.

### [Hemiasterlin]

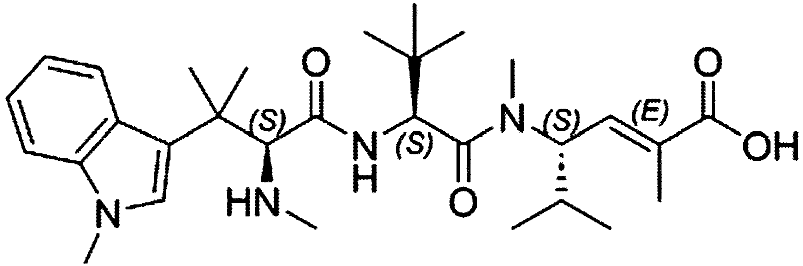

In the present specification, "isomer" refers to a relationship between compounds having the same chemical formula but not necessarily identical properties. Types of such isomers include structural isomers and stereoisomers. The "stereoisomer" refers to compounds having the same chemical constitution but differing in the spatial arrangement of atoms or groups, and includes not only optical isomers (for example, essentially pure enantiomers, essentially pure diastereomers, or mixtures thereof), but also conformational isomers (i.e., isomers differing only in the angle of one or more chemical bonds), positional isomers (in particular, tautomers), or geometric isomers (e.g., cis-trans isomers). In the present specification, the isomers of the compound of Chemical Formula 1 means stereoisomers, but are not limited thereto.

The compound of Chemical Formula 1 according to the present invention is specifically selected from the following compounds, and may be in the form of each isomeric compound or a mixture thereof.

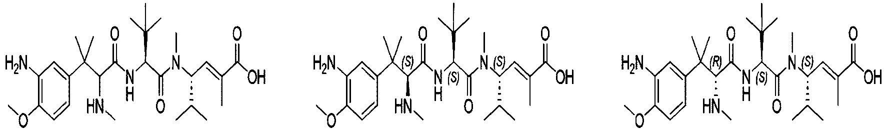

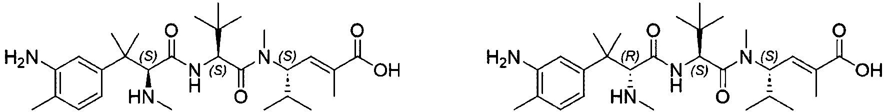

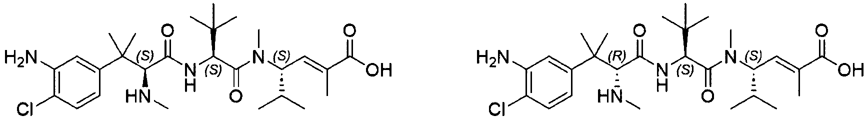

In the present specification, "salt" refers to a salt according to one aspect of the present disclosure having the desired pharmacological activity of the parent compound, which may be a salt that does not cause severe irritation to the organism to which the compound is applied and does not impair the biological activity and physicochemical properties of the compound. Examples include salts of inorganic ions such as sodium, potassium, calcium, magnesium, lithium, copper, manganese, zinc, and iron, as well as salts of inorganic acids such as hydrochloric acid, phosphoric acid, and sulfuric acid. Other examples include salts of organic acids such as ascorbic acid, citric acid, tartaric acid, lactic acid, maleic acid, malonic acid, fumaric acid, glycolic acid, succinic acid, propionic acid, acetic acid, orotic acid, and acetylsalicylic acid, and amino acid salts such as lysine, arginine, and guanidine.

When the composition of the present specification is particularly a pharmaceutical composition, the "salt" may be a "pharmaceutically acceptable salt." The term "pharmaceutically acceptable" means that, when used at a conventional medicinal dosage, the salt can avoid significant toxic effects and can obtain, or has obtained, approval from a government or an equivalent regulatory authority for use in animal, and more specifically in humans, or is listed in a pharmacopoeia or otherwise generally recognized in the art.

In the present specification, the "salt" or "pharmaceutically acceptable salt" refers to addition salts of inorganic acid salts, organic acid salts, or metal salts of the compound. The inorganic acid salts may be hydrochloride, bromide, phosphate, sulfate, or bisulfate. The organic acid salts may be formate, acetate, propionate, lactate, oxalate, tartrate, malate, maleate, citrate, fumarate, besylate, camsylate, edisylate, trichloroacetate, trifluoroacetate, benzoate, gluconate, methanesulfonate, glycolate, succinate, 4-toluenesulfonate, galacturonate, embonate, glutamate, methanesulfonate, ethanesulfonate, benzenesulfonate, p-toluenesulfonate or aspartate. The metal salts may be calcium salts, sodium salts, magnesium salts, strontium salts or potassium salts.

In the present specification, "solvate" refers to a compound solvated with an organic or inorganic solvent. The solvate in the present specification is, for example, a hydrate.

The compound(Chemical Formula 1) of the present invention may be provided in a form conjugated to a carrier.

In the present invention, "carrier" means a substance capable of selectively and specifically delivering the compound according to the invention to a target site, for example, to cancer cells, and may be an antibody, peptide, repebody and/or aptamer, but is not limited thereto, and is preferably an antibody.

For example, the carrier of the present invention is an antibody, peptide, repebody, or aptamer that specifically binds to one or more substances (antigens) selected from the group consisting of 4-1BB, 5T4, integrin, Activin, amyloid beta, angiopoietin (angiopoietin 1 or 2), angiopoietin-like protein 3, B-cell maturation antigen (BCMA), B-cell activating factor (BAFF), B7-H3, complement 5, CCR4, CCR5, CCL11, CD2, CD3, CD4, CD6, CD11a, CD16A, CD19, CD20, CD22, CD25, CD27, CD28, CD30, CD32B, CD33, CD38, CD40, CD45, CD46, CD47, CD52, CD56, CD62, CD70, CD73, CD74, CD79b, CD80, CD105, CD123, CD154, CD166, CD262, CD278, CD319, CD326, carcinoembryonic antigen (CEA), CGRP, Claudin-18, c-Met, CSF-1, CSF-1 receptor, CTLA4, DLL3, EGF receptor, hemophilia factor, Fc receptor, FGF23, folate receptor, GD2, glucocorticoid-induced TNF receptor (GITR), Glypican 3, GM-CSF, HER2, HER3, TROP2, hepatocyte growth factor (HGF), interferon receptor, interferon gamma, IgE, IGF-1 receptor, interleukin-1, interleukin-2 receptor, interleukin-4, interleukin-4 receptor, interleukin-5, interleukin-5 receptor, interleukin-6, interleukin-6 receptor, interleukin-8, interleukin-12/23, interleukin-13, interleukin-17A, interleukin-17 receptor A, interleukin-23, interleukin-31 receptor, interleukin-36 receptor, lymphocyte-activation gene 3 (LAG3), lysyl oxidase homolog 2 (LOXL2), mesothelin, mucin-1, mucin-16, Nectin-4, nerve growth factor (NGF), OX40, proprotein convertase subtilisin/kexin type 9 (PCSK9), PD-1, PD-L1, phospholipase C, RANKL (receptor activator of nuclear factor kappa-B ligand), tyrosine-protein kinase transmembrane receptor (ROR1), sialic acid-binding Ig-like lectin 15 (Siglec-15), transforming growth factor beta (TGF-β), TIGIT (T-cell immunoreceptor with immunoglobulin and ITIM domain), T-cell immunoglobulin and mucin-domain containing-3 (Tim-3), tissue factor, tissue factor pathway inhibitor (TFPI), TORP-2, tumor necrosis factor (TNF), thymic stromal lymphopoietin (TSLB), colony stimulating factor 1 receptor (CSF1R), vascular endothelial growth factor (VEGF), VEGF receptor, and von Willebrand Factor (vWF).

In the present invention, non-limiting examples of the antibody may be Urelumab, Utomilumab, Bebtelovimab, Aducanumab, Bapinezumab, Crenezumab, Donanemab, Gantenerumab, Lecanemab, Solanezumab, Nesvacumab, Evinacumab, Enoblituzumab, Omburtamab, Belimumab, Ianalumab, Tabalumab, Bertilimumab, Mogamulizumab, Leronlimab, Siplizumab, Foralumab, Muromonab-CD3, Otelixizumab, Teplizumab, Ibalizumab, Tregalizumab, Zanolimumab, Itolizumab, Efalizumab, Inebilizumab, Tafasitamab, Tositumomab, Ocrelizumab, Ofatumumab, Rituximab, Ublituximab, Veltuzumab, Epratuzumab, Basiliximab, Daclizumab, Varlilumab, Lulizumab, Iratumumab, Lintuzumab, Daratumumab, Felzartamab, Isatuximab, Mezagitamab, Bleselumab, Dacetuzumab, Iscalimab, Lucatumumab, Mitazalimab, Sotigalimab, Dapirolizumab, Apamistamab, Ligufalimab, Magrolimab, Alemtuzumab, Crizanlizumab, Inclacumab, Cusatuzumab, Oleclumab, Milatuzumab, Galiximab, Carotuximab, Adecatumumab, Eptinezumab, Erenumab, Fremanezumab, Galcanezumab, Zolbetuximab, Onartuzumab, Eculizumab, Pozelimab, Ravulizumab, Lacnotuzumab, Axatilimab, Cabiralizumab, Emactuzumab, Ipilimumab, Quavonlimab, Tremelimumab, Zalifrelimab, Cetuximab, Depatuxizumab, Futuximab, Imgatuzumab, Matuzumab, Modotuximab, Necitumumab, Nimotuzumab, Panitumumab, Tomuzotuximab, Zalutumumab, Batoclimab, Nipocalimab, Rozanolixizumab, Burosumab, Farletuzumab, Dinutuximab, Naxitamab, Ragifilimab, Gimsilumab, Lenzilumab, Mavrilimumab, Namilumab, Otilimab, Plonmarlimab, Codrituzumab, Margetuximab, Pertuzumab, Trastuzumab, Datopotamab, Patritumab, Seribantumab, Duligotuzumab, Ficlatuzumab, Rilotumumab, Alomfilimab, Anifrolumab, Emapalumab, Ligelizumab, Omalizumab, Cixutumumab, Dalotuzumab, Figitumumab, Ganitumab, Teprotumumab, Bermekimab, Canakinumab, Gevokizumab, Briakinumab, Ustekinumab, Anrukinzumab, Cendakimab, Lebrikizumab, Tralokinumab, Brodalumab, Bimekizumab, Ixekizumab, Secukinumab, Brazikumab, Guselkumab, Mirikizumab, Risankizumab, Tildrakizumab, Nemolizumab, Imsidolimab, Spesolimab, Pascolizumab, Dupilumab, Depemokimab, Mepolizumab, Reslizumab, Benralizumab, Clazakizumab, Olokizumab, Siltuximab, Sirukumab, Ziltivekimab, Levilimab, Sarilumab, Satralizumab, Tocilizumab, Abituzumab, Favezelimab, Fianlimab, Ieramilimab, Relatlimab, Simtuzumab, Abagovomab, Oregovomab, Tanezumab, Ivuxolimab, Rocatinlimab, Tavolimab, Telazorlimab, Vonlerolizumab, Alirocumab, Bococizumab, Ebronucimab, Evolocumab, Frovocimab, Ongericimab, Tafolecimab, Dostarlimab, Balstilimab, Camrelizumab, Cemiplimab, Geptanolimab, Nivolumab, Pembrolizumab, Penpulimab, Pidilizumab, Prolgolimab, Retifanlimab, Sasanlimab, Serplulimab, Sintilimab, Spartalizumab, Tislelizumab, Toripalimab, Ezabenlimab, Zimberelimab, Atezolizumab, Avelumab, Cosibelimab, Sugemalimab, Durvalumab, Envafolimab, Suvratoxumab, Denosumab, Zilovertamab, Elotuzumab, Domvanalimab, Etigilimab, Ociperlimab, Tiragolumab, Vibostolimab, Surzebiclimab, Cobolimab, Sabatolimab, Concizumab, Marstacimab, Adalimumab, Golimumab, Infliximab, Certolizumab, Conatumumab, Tigatuzumab, Tezepelumab, Gatipotuzumab, Cabiralizumab, Bevacizumab, Brolucizumab, Ranibizumab, Olinvacimab, Icrucumab, Ramucirumab, Caplacizumab, Abrilumab, Etrolizumab, Vedolizumab, Intetumumab, Natalizumab, Obrindatamab, Elranatamab, Linvoseltamab, Teclistamab, Epcoritamab, Glofitamab, Mosunetuzumab, Odronextamab, Flotetuzumab, Vibecotamab, Catumaxomab, Cibisatamab, Talquetamab, Ubamatamab, Emfizatamab, Blinatumomab, Amivantamab, Emicizumab, Zenocutuzumab, Zanidatamab, Tibulizumab, Naptumomab, Belantamab, Pivekimab, Praluzatamab, Coltuximab, Denintuzumab, Loncastuximab, Ibritumomab, Inotuzumab, Epratuzumab, Moxetumomab, Brentuximab, Gemtuzumab, Vadastuximab, Lorvotuzumab, Polatuzumab, Tusamitamab, Telisotuzumab, Rovalpituzumab, Depatuxizumab, Farletuzumab, Mirvetuximab, Disitamab, Anetumab, Enfortumab, Sacituzumab, Vobarilizumab, Cadonilimab, Vudalimab, Tebotelimab, Ivonescimab, Erfonrilimab, Ozoralizumab, Faricimab, Vanucizumab or Navicixizumab, but is not limited thereto.

The present invention provides a pharmaceutical composition for preventing or treating cancer, comprising a compound of Chemical Formula 1 or a carrier-drug conjugate. The pharmaceutical composition of the present invention may be administered in a therapeutically effective amount to a subject in need thereof.

In the present invention, the cancer includes pseudomyxoma, intrahepatic cholangiocarcinoma, hepatoblastoma, liver cancer, thyroid cancer, colon cancer, testicular cancer, myelodysplastic syndrome, glioblastoma, oral cancer, lip cancer, mycosis fungiodes, acute myeloid leukemia, acute lymphoblastic leukemia, basal cell carcinoma, ovarian epithelial carcinoma, ovarian germ cell carcinoma, male breast cancer, brain cancer, pituitary adenoma, multiple myeloma, gallbladder cancer, bile duct cancer, colorectal cancer, chronic myelogenous leukemia, chronic lymphocytic leukemia, retinoblastoma, choroidal melanoma, ampulla of Vater cancer, bladder cancer, peritoneal cancer, parathyroid cancer, adrenal cancer, paranasal sinus cancer, non-small cell lung cancer, tongue cancer, astrocytoma, small cell lung cancer, pediatric brain cancer, pediatric lymphoma, childhood leukemia, small bowel cancer, meningioma, esophageal cancer, glioma, renal pelvis cancer, kidney cancer, heart cancer, duodenal cancer, malignant soft tissue cancer, malignant bone cancer, malignant lymphoma, malignant mesothelioma, malignant melanoma, eye cancer, vulvar cancer, ureteral cancer, urethral cancer, unknown primary neoplasm, gastric lymphoma, gastric cancer, gastric carcinoma, gastrointestinal stromal cancer, Wilms' cancer, breast cancer, Triple-negative breast cancer(TNBC), sarcoma, penile cancer, pharyngeal cancer, gestational trophoblastic disease, cervical cancer, endometrial cancer, uterine sarcoma, prostate cancer, metastatic bone cancer, metastatic brain cancer, mediastinal cancer, rectal cancer, occipital carcinoid tumors, vaginal cancer, spinal cancer, vestibular schwannoma, pancreatic cancer, salivary gland cancer, Kaposi's sarcoma, Paget's disease, tonsil cancer, squamous cell carcinoma, lung adenocarcinoma, lung cancer, squamous cell lung cancer, skin cancer, anal cancer, rhabdomyosarcoma, laryngeal cancer, pleural cancer, blood cancer, and thymic carcinoma.

In the present specification, the term "prevention" means any act of suppressing the onset of cancer symptoms or delaying the development of cancer by administration of the pharmaceutical composition. The term "treatment" means any act of improving or favorably modifying the symptoms of cancer by administration of the pharmaceutical composition.

In the present specification, "patient," "subject," and "individual" refer to an animal such as a mammal. In certain embodiments, the patient is a human. In other embodiments, the patient is a non-human animal such as a dog, cat, livestock (e.g., horse, pig, or donkey), chimpanzee, or monkey.

As used in the present invention, the term "therapeutically effective amount" refers to an amount of the compound or carrier-drug conjugate effective for the treatment or prevention of cancer. Specifically, a "therapeutically effective amount" means an amount sufficient to treat a disease with a reasonable benefit/risk ratio applicable to medical treatment, wherein the effective dose level may be determined according to the type of subject and severity of the condition, age, sex, and type of disease, activity of the drug, sensitivity to the drug, timing and route of administration and excretion rate, duration of treatment, concomitant drugs, and other factors well known in the art. The pharmaceutical composition of the present invention may be administered as a monotherapy or in combination with other therapeutic agents, and may be administered sequentially or simultaneously with marketed therapeutic agents, and may be administered as a single or multiple dose. Considering all of these factors, it is important to administer an amount that achieves the maximum effect with the minimum dose without side effects. Since the compound of Chemical Formula 1 of the present invention, its derivatives, isomers, or pharmaceutically acceptable salts, or the carrier-drug conjugate comprising thereof, exhibits a dose-dependent effect, the dosage can be readily determined by those skilled in the art depending on various factors such as the patient's condition, age, sex, and complications. Because the active ingredient of the pharmaceutical composition of the present invention has excellent safety, it may also be used at a dose higher than the determined administration dose.

Hereinafter, the synthesis of the compounds of the present invention and the effects thereof are specifically confirmed through Examples.

### [Example 1]

### Synthesis of Compounds of Chemical Formula 2 and 3

### 1-1. Synthesis of A fragment

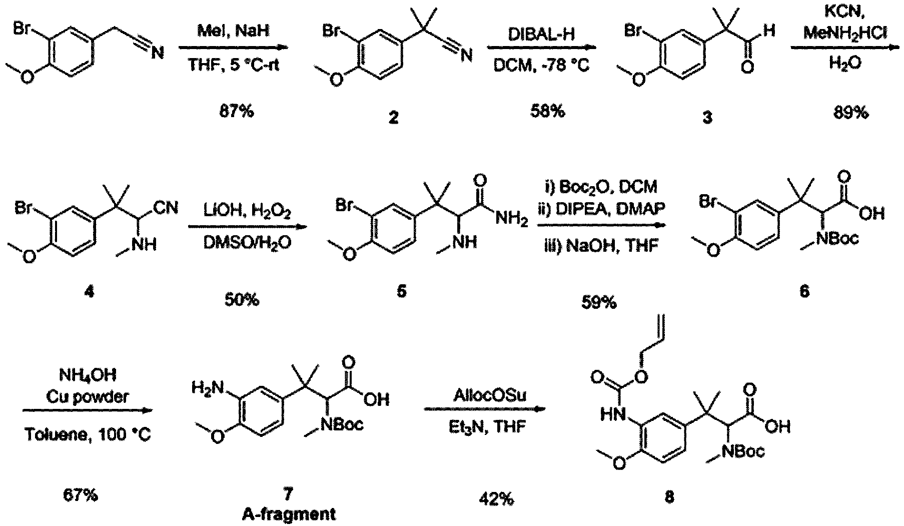

In the chemical structure, Boc₂O refers to Di-tert-butyl dicarbonate.

### Step 1:

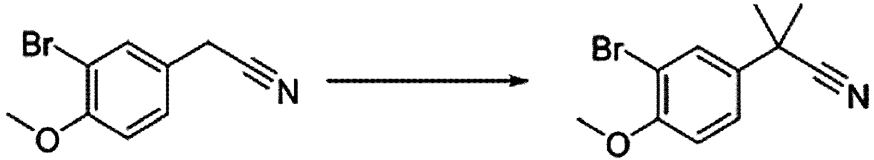

To a solution of 2-(3-bromo-4-methoxyphenyl)acetonitrile (5.00 g, 1 eq, 22.1 mmol) in dry THF (30 mL) was added methyl iodide (7.53 g, 3.30 mL, 2.4 eq, 53.1 mmol). The reaction mixture was cooled to 4 °C, and sodium hydride (60% dispersion in mineral oil, 2.12 g, 60% Wt, 2.4 eq, 53.1 mmol) was added portionwise. After the addition was completed, the reaction mixture was warmed to room temperature and stirred overnight. Additional methyl iodide (628 mg, 277 µL, 0.2 eq, 4.42 mmol) and sodium hydride (60% dispersion in mineral oil, 177 mg, 60% Wt, 0.2 eq, 4.42 mmol) were added, and the reaction mixture was stirred for 1 hour at room temperature. The reaction mixture was poured into ice water (~100 mL) and extracted with EtOAc (3×). The combined organic layers were washed with brine, dried over Na₂SO₄, and concentrated under reduced pressure to afford a brown oil (6.47 g). The crude product was purified by flash column chromatography (120 g SiO₂, 0-20% EtOAc in heptane), and the product fractions were concentrated under reduced pressure to yield a colorless oil.

Yield: 4.9 g, 87%.

¹H NMR (400 MHz, CDCl₃) δ 7.62 (d, J = 2.6 Hz, 1H), 7.40 (dd, *J* = 8.6, 2.5 Hz, 1H), 6.90 (d, *J* = 8.7 Hz, 1H), 3.89 (s, 3H), 1.70 (s, 6H). m/z 253.2/255.2 [M+H]⁺, Br isotope pattern.

### Step 2:

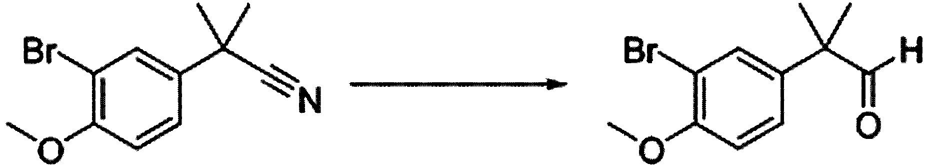

A solution of 2-(3-bromo-4-methoxyphenyl)-2-methylpropanenitrile (4.68 g, 1 eq, 18.4 mmol) in dry DCM (40 mL) was cooled to -78 °C. Diisobutylaluminum hydride (1 M in hexane, 3.40 g, 23.9 mL, 1.3 eq, 23.9 mmol) was added dropwise to the solution within 15 minutes. The reaction mixture was stirred at -78 °C for 1.5 hours. Additional diisobutylaluminum hydride (1 M in hexane, 655 mg, 4.60 mL, 0.25 eq, 4.60 mmol) was added dropwise, and the mixture was allowed to stir at -78 °C for 30 minutes. The reaction mixture was then warmed to 0 °C, quenched slowly with 2 M HCI (60 mL), stirred at 0 °C for 30 minutes, and then stirred at room temperature for another 30 minutes. The mixture was diluted with water and extracted with DCM (3×). The combined organic layers were washed with brine, dried over Na₂SO₄, and concentrated under reduced pressure to afford a solid (4.39 g). The crude product was suspended in DCM and filtered. The filtrate was purified by column chromatography (120 g SiO₂, 0-20% EtOAc in heptane), and the product fractions were concentrated under reduced pressure to give a light-yellow oil (2.77 g, 58%).

¹H NMR (400 MHz, CDCl₃) δ 9.44 (s, 1H), 7.46 (d, *J* = 2.4 Hz, 1H), 7.16 (dd, *J* = 8.6, 2.4 Hz, 1H), 6.90 (d, *J* = 8.6 Hz, 1H), 3.89 (s, 3H), 1.44 (s, 6H). m/z 257.2/259.2 [M+H]⁺, Br isotope pattern.

### Step 3:

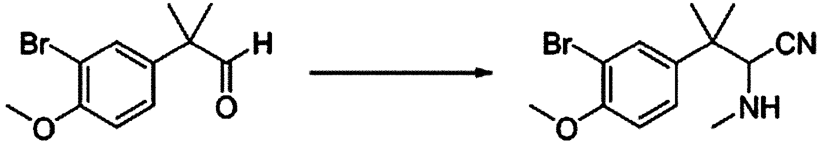

A 100 mL round-bottom flask was charged with potassium cyanide (0.56 g, 1 eq, 8.6 mmol) and water (13 mL). The solution was cooled to 0 °C, and methylamine hydrochloride (0.58 g, 1 eq, 8.6 mmol) was added. A solution of 2-(3-bromo-4-methoxyphenyl)-2-methylpropanal (2.2 g, 1 eq, 8.6 mmol) in methanol (13 mL) was then added. The resulting white suspension was stirred at room temperature overnight. The reaction mixture was diluted with H₂O (40 mL) and extracted with CH₂Cl₂ (3 × 50 mL). After combining the organic layers, the mixture was washed with brine (20 mL), and dried over Na₂SO₄, and the solvent was removed under vacuum to afford 3-(3-bromo-4-methoxyphenyl)-3-methyl-2-(methylamino)butanenitrile as a colorless oil.

Yield: 2.56 g, 89%.

¹H NMR (400 MHz, CDCl3) δ 7.57 (d, *J* = 2.4 Hz, 1H), 7.36 (dd, *J* = 8.7, 2.4 Hz, 1H), 6.89 (d, *J* = 8.7 Hz, 1H), 3.89 (s, 3H), 3.45-3.29 (m, 1H), 2.48 (s, 3H), 1.50 (d, *J* = 11.5 Hz, 6H). m/z 297.0/299.0 [M+H]⁺, Br isotope pattern.

### Step 4:

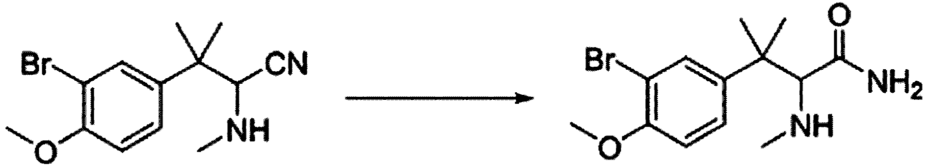

A solution of 3-(3-bromo-4-methoxyphenyl)-3-methyl-2-(methylamino)butanenitrile (2.53 g, 1 eq, 8.51 mmol) in DMSO (45 mL) was treated with lithium hydroxide monohydrate 1M (1.71 g, 40.9 mL, 1.0 M, 4.8 eq, 40.9 mmol) in water and hydrogen peroxide (4.63 g, 4.17 mL, 30% w/w, 4.8 eq, 40.9 mmol). The reaction mixture was stirred at room temperature for 2 h. After dilution with water (385 mL), the mixture was extracted with ethyl acetate (2 × 385 mL). The organic layers were washed with water and brine. After drying the organic layers over Na₂SO₄, the organic layers concentrated under reduced pressure to give a colorless sticky solid (2.8 g). Purification by flash column chromatography (80 g SiO₂, 0-5% MeOH in dichloromethane) afforded the product as a white foam.

Yield: 1.35 g (50%).

¹H NMR (400 MHz, DMSO): δ 7.50 (d, *J* = 2.4 Hz, 1H), 7.35-7.23 (m, 2H), 7.05-6.97 (m, 2H), 3.81 (s, 3H), 2.99-2.90 (m, 1H), 2.07 (s, 3H), 1.44 (bs, 1H), 1.25 (d, J = 7.8 Hz, 6H). MS (ESI): m/z 315.2/317.2 [M+H]⁺, Br isotope pattern.

### Step 5:

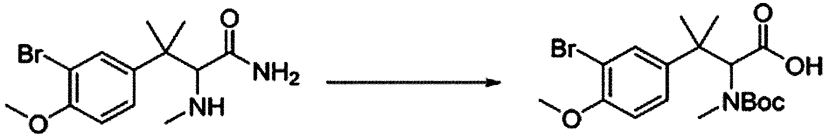

dichloromethane (15 mL) was added to 3-(3-bromo-4-methoxyphenyl)-3-methyl-2-(methylamino)butanamide (1.31 g, 1 eq, 4.16 mmol). Subsequently, Boc₂O (3.17 g, 3.5 eq, 14.5 mmol) was added. After several minutes, a clear and colorless solution was obtained. The reaction mixture was stirred at room temperature for 3 days. DIPEA (591 mg, 787 µL, 1.1 eq, 4.57 mmol) and DMAP (50.8 mg, 0.1 eq, 416 µmol) were then added. The reaction mixture was stirred at room temperature for 2 h. Water (45 mL) was added and the mixture was extracted with heptane (3 × 50 mL). The organic layer was washed with water (25 mL) and brine (25 mL). The organic layer was dried over sodium sulfate and concentrated to dryness. A yellow oil (2.8 g) was obtained, and dissolved in THF (6.5 mL). Sodium hydroxide, 5 M (831 mg, 4.16 mL, 5.0 molar, 5 eq, 20.8 mmol), was added, and the reaction mixture was stirred at room temperature for 2 h. Thereafter, water (25 mL) was added. The mixture was filtered, and the residue was washed with 0.5 M NaOH (25 mL). THF was removed under reduced pressure. The aqueous solution was washed with heptane, neutralized with solid citric acid, and extracted with ethyl acetate (3×). The organic layer was washed with brine, dried over sodium sulfate, and concentrated under reduced pressure to afford a colorless oil (1.37 g). Purification by column chromatography (40 g SiO₂, 0-50% ethyl acetate in heptane) and concentration of the product fractions under reduced pressure gave the product as a white foam.

Yield: 1.02 g, 59%.

¹H NMR indicated that the target compound was a mixture of rotamers.

1H NMR (400 MHz, CDCl₃) δ 7.60-7.51 (m, 1H), 7.38-7.27 (m, 1H), 6.83 (d, J = 8.7 Hz, 1H), 5.07-4.84 (m, 1H), 3.88 (s, 3H), 2.85-2.62 (m, 3H), 1.58-1.39 (m, 15H). m/z 414.2/416.2 [M-H]⁻, Br isotope pattern.

### Step 6:

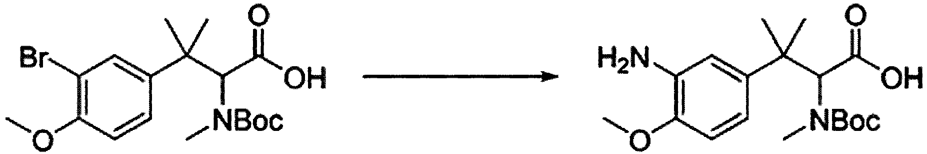

A mixture of 3-(3-bromo-4-methoxyphenyl)-2-((tert-butoxycarbonyl)(methyl)amino)-3-methylbutanoic acid (350 mg, 1 eq, 841 µmol) and toluene (5.0 mL) in a 20 mL microwave vial was charged with ammonium hydroxide solution, 32% (9.21 g, 10.2 mL, 32% Wt, 100 eq, 84.1 mmol), followed by addition of copper powder (160 mg, 3 eq, 2.52 mmol). The vial was capped and the reaction mixture was stirred at 100 °C for 6 hours. The reaction mixture was filtered, and the filtrate was evaporated to dryness. The residue was co-evaporated with ethanol (3x) to remove water. The crude material was coated onto hydromatrix and purified by flash column chromatography (12 g SiO₂, 0-10% methanol in dichloromethane). The product fractions were concentrated under reduced pressure to afford 232 mg of a green oil/foam. The product was directly used in the next step.
Yield: 67%.
¹H NMR (400 MHz, CDCl₃) δ 7.23-5.96 (m, 3H), 5.20-4.70 (m, 1H), 4.05-3.55 (m, 3H), 3.01-2.41 (m, 4H), 1.93-1.09 (m, 17H). m/z 705.2 [2M+H]⁺

### Step 7:

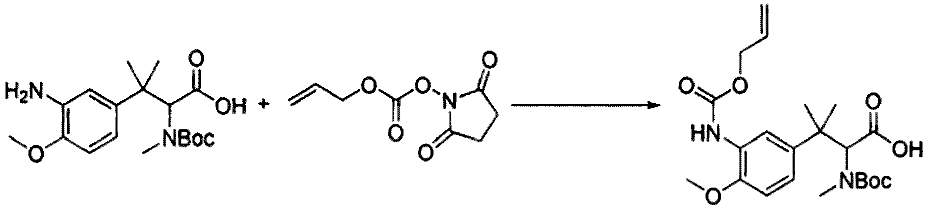

Alloc-OSu (232 mg, 2 eq, 1.16 mmol) was added to a solution of 3-(3-amino-4-methoxyphenyl)-2-((tert-butoxycarbonyl)(methyl)amino)-3-methylbutanoic acid (205 mg, 1 eq, 582 µmol) in tetrahydrofuran (1.5 mL), and then triethylamine (177 mg, 243 µL, 3 eq, 1.75 mmol) was added. The reaction mixture was stirred at room temperature overnight. The solvent was removed under reduced pressure, and the residue was purified by flash column chromatography (12g SiO₂, 0-10% methanol in dichloromethane). The product fractions were concentrated to afford 143 mg (42%) of a light brown oil. The NMR spectrum indicated rotamers.
¹H NMR (400 MHz, CDCl₃) δ 8.20 (s, 1H), 7.24 (s, 1H), 7.11-6.97 (m, 1H), 6.79 (d, *J* = 8.6 Hz, 1H), 6.05-5.92 (m, 1H), 5.41-5.33 (m, 1H), 5.28-5.23 (m, 1H), 4.94 (s, 1H), 4.70-4.64 (m, 2H), 3.84 (s, 3H), 2.77 (s, 1H), 2.65 (s, 2H), 1.56-1.40 (m, 16H). m/z 459.2 [M+Na]⁺

### 1-2. Synthesis of the BCD Fragment

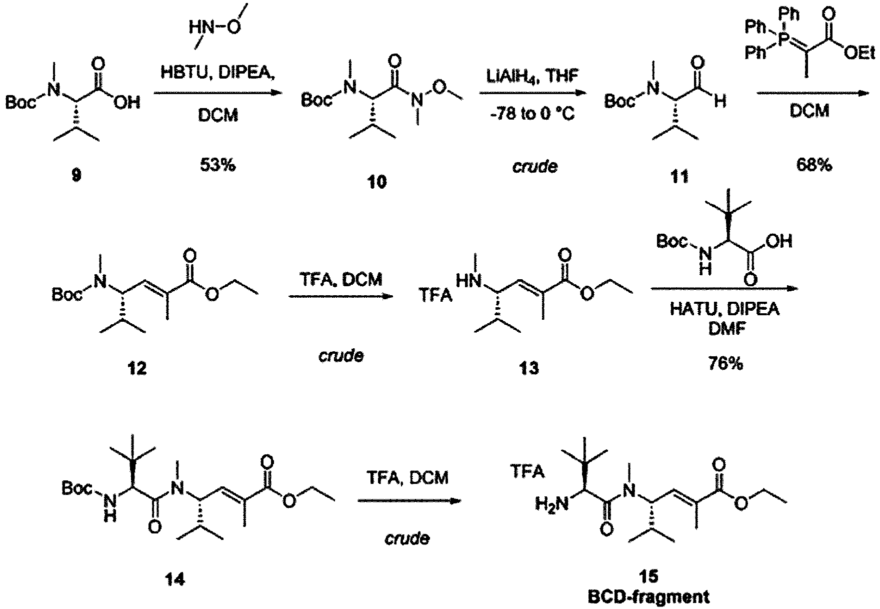

### Step 1:

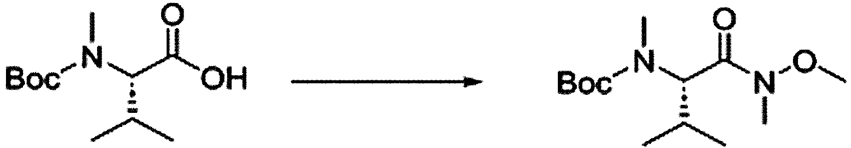

To a stirred solution of N-(tert-butoxycarbonyl)-N-methyl-L-valine (5.0 g, 1 eq, 22 mmol), N,O-dimethylhydroxylamine hydrochloride (2.3 g, 1.1 eq, 24 mmol) and DIPEA (5.6 g, 7.5 mL, 2 eq, 43 mmol) in dichloromethane (50 mL) was slowly added HBTU (9.0 g, 1.1 eq, 24 mmol). The reaction mixture was stirred at room temperature overnight. The reaction mixture was washed with water, and the organic layer was separated and dried over Na₂SO₄. The organic layer was then evaporated to dryness and purified by column chromatography (120 g SiO₂, 0-40% ethyl acetate in heptane). The product was obtained as a transparent colorless oil.

Yield: 3.15 g (53%).

¹H NMR (400 MHz, DMSO-d₆) mixture of rotamers δ 4.94-4.44 (m, 1H), 3.64 (d, *J* = 10.0 Hz, 3H), 3.19-3.01 (m, 3H), 2.74-2.60 (m, 3H), 2.21-2.04 (m, 1H), 1.48-1.33 (m, 9H), 0.89-0.73 (m, 6H).

### Step 2:

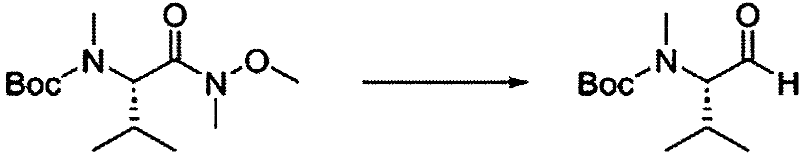

At -78 °C, a solution of lithium aluminium hydride 2.4 M in THF (69.2 mg, 759 µL, 1 eq, 1.82 mmol) was added in one portion to a solution of tert-butyl (S)-(1-(methoxy(methyl)amino)-3-methyl-1-oxobutan-2-yl)(methyl)carbamate (500 mg, 1 eq, 1.82 mmol) in THF (2.5 mL). The reaction mixture was then warmed to 0 °C and stirred for 15 min. The reaction mixture was poured into a stirred ice-cold aqueous solution of potassium bisulfate (0.25 M, 496 mg, 14.6 mL, 2 eq, 3.64 mmol), and the layers were separated. The aqueous layer was extracted with diethyl ether (3×). The combined organic layers were washed with 1 M HCI (2×), saturated aqueous NaHCO₃ (2×) and brine, then dried over Na₂SO₄, and concentrated (bath temperature below 20 °C). The crude aldehyde was obtained and used directly in the next step without purification.

### Step 3:

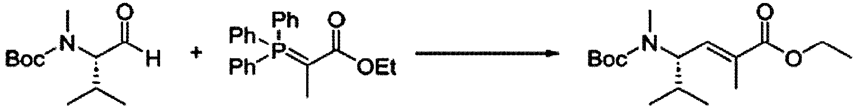

A solution of tert-butyl (S)-methyl(3-methyl-1-oxobutan-2-yl)carbamate (392 mg, 1 eq, 1.82 mmol) in dichloromethane (2.5 mL) was treated with ethyl 2-(triphenyl-λ⁵-phosphaneylidene)propanoate (857 mg, 1.3 eq, 2.37 mmol), and the reaction mixture was stirred at room temperature overnight. The mixture was diluted with water and extracted with diethyl ether (3×). The combined organic layers were washed with brine, dried over Na₂SO₄, and concentrated to give a yellow oil (1.0 g). The crude product was purified by flash column chromatography (40 g SiO₂, 0-10% ethyl acetate in heptane) to afford the desired product as a colorless oil (237 mg).

¹H NMR (400 MHz, CDCl₃): δ 6.70-6.60 (m, 1H), 4.64-4.25 (m, 1H), 4.21 (q, *J* = 7.1 Hz, 2H), 2.80-2.62 (m, 3H), 1.96-1.81 (m, 4H), 1.45 (s, 9H), 1.31 (t, *J* = 7.0 Hz, 3H), 0.96-0.79 (m, 6H). MS (ESI): m/z 322.2 [M+Na]⁺.

### Step 4:

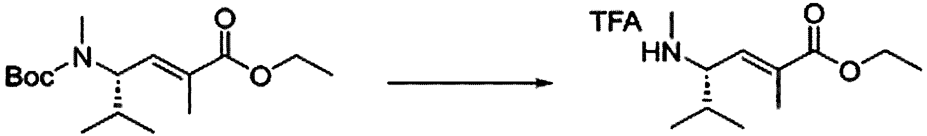

A solution of ethyl (S,E)-4-((tert-butoxycarbonyl)(methyl)amino)-2,5-dimethylhex-2-enoate (235 mg, 1 eq, 785 µmol) in dichloromethane (2.5 mL) was treated with trifluoroacetic acid (2.5 mL). The reaction mixture was stirred at room temperature for 30 minutes. The solvent was removed under reduced pressure, and the residue was co-evaporated with dichloromethane (6×) to afford a slightly yellow turbid oil (325 mg). The product was used directly in the next step.

¹H NMR (400 MHz, CDCl₃): δ 8.86 (d, *J* = 174.6 Hz, 1H), 6.63-6.56 (m, 1H), 4.33-4.17 (m, 2H, coincides with H₂O peak), 3.82-3.69 (m, 1H), 2.62 (s, 3H), 2.25-2.13 (m, 1H), 1.95 (s, 3H), 1.33 (t, *J* = 7.1 Hz, 3H), 1.02 (dd, *J* = 12.2, 6.8 Hz, 6H). m/z 200.0 [M+H]⁺.

### Step 5:

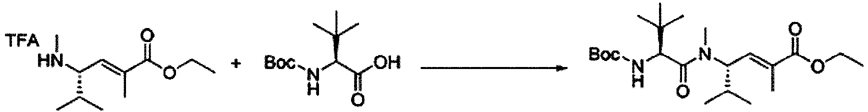

To a solution of N-Boc-L-*tert*-leucine (272 mg, 1.5 eq, 1.18 mmol) and HATU (448 mg, 1.5 eq, 1.18 mmol) in N,N-dimethylformamide (3.0 mL) was added DIPEA (101 mg, 137 µL, 1 eq, 785 µmol). The mixture was stirred for 5 minutes, after which a solution of ethyl (S,E)-2,5-dimethyl-4-(methylamino)hex-2-enoate 2,2,2-trifluoroacetate (246 mg, 1 eq, 785 µmol) and DIPEA (507 mg, 684 µL, 5 eq, 3.93 mmol) in peptide-grade N,N-dimethylformamide (1.5 mL) was added dropwise. The mixture was stirred at room temperature for 90 minutes. The reaction mixture was diluted with ethyl acetate, washed with water (2×) and brine (2×), dried over sodium sulfate, and concentrated to give a yellow oil (478 mg). Purification by flash column chromatography (24 g SiO₂, 0-50% ethyl acetate in heptane) afforded the product fractions, which were concentrated to give the title compound as a colorless oil (272 mg, 76%).
¹H NMR (400 MHz, CDCl₃): δ 6.68-6.60 (m, 1H), 5.22 (d, *J* = 10.1 Hz, 1H), 5.15-5.06 (m, 1H), 4.43 (d, *J* = 10.1 Hz, 1H), 4.20 (q, *J* = 7.1 Hz, 2H), 2.99 (s, 3H), 1.90 (m, 4H), 1.42 (s, 9H), 1.31 (t, *J* = 7.1 Hz, 3H), 0.96 (s, 9H), 0.86 (dd, *J* = 18.3, 6.6 Hz, 6H). m/z 413.2 [M+H]⁺

### Step 6:

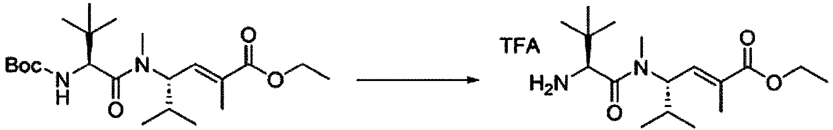

To a solution of ethyl (S,E)-4-((S)-2-((tert-butoxycarbonyl)amino)-N,3,3-trimethylbutanamido)-2,5-dimethylhex-2-enoate (260 mg, 90.7% Wt, 1 eq, 572 µmol) in dichloromethane (4.0 mL) was added trifluoroacetic acid (4.0 mL). The reaction mixture was stirred at room temperature for 30 minutes. The solvent was removed under reduced pressure, and the residue was co-evaporated with dichloromethane (6×) to afford the title compound as a colorless oil (329 mg).
¹H NMR (400 MHz, CDCl₃): δ 7.92 (bs, 2H), 6.69-6.62 (m, 1H), 5.70 (bs, 3H), 5.00 (t, *J* = 10.1 Hz, 1H), 4.27 (s, 1H), 4.22 (q, *J* = 7.1 Hz, 2H), 2.98 (s, 3H), 2.03-1.93 (m, 1H), 1.91 (s, 3H), 1.31 (t, *J* = 7.1 Hz, 3H), 1.08 (s, 9H), 0.87 (dd, *J* = 11.3, 6.5 Hz, 6H). MS (ESI): m/z 313.4 [M+H]⁺

### 1-3. Synthesis of Compound of Chemical Formula 2 and 3

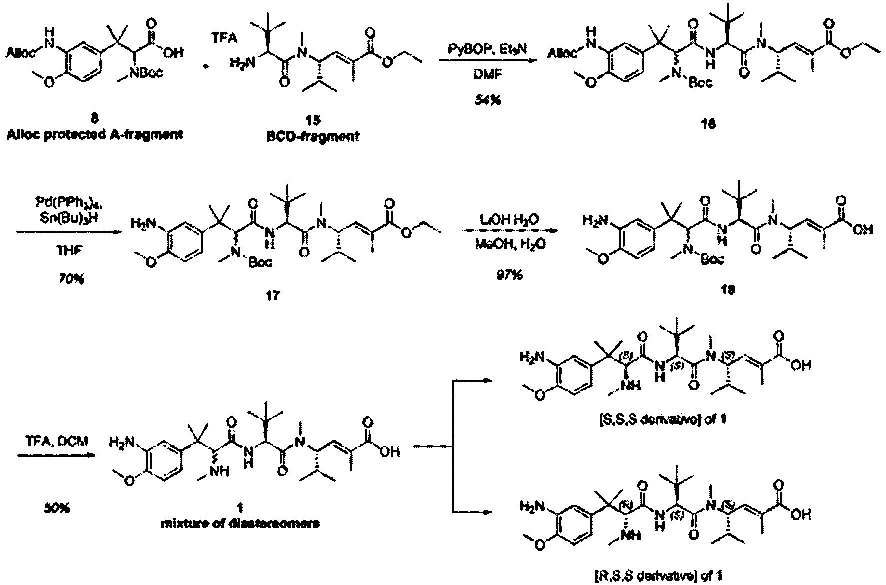

### Step 1:

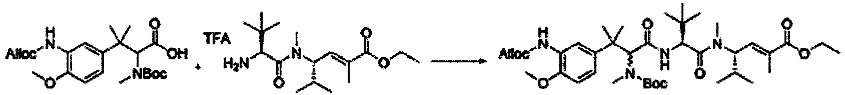

A solution of 3-(3-(((allyloxy)carbonyl)amino)-4-methoxyphenyl)-2-((tert-butoxycarbonyl)(methyl)amino)-3-methylbutanoic acid [A fragment] (128 mg, 85% Wt, 1 eq, 249 µmol) in N,N-dimethylformamide (1.8 mL) was added with ethyl (S,E)-4-((S)-2-amino-N,3,3-trimethylbutanamido)-2,5-dimethylhex-2-enoate 2,2,2-trifluoroacetate (142 mg, 75% Wt, 1 eq, 249 µmol), and subsequently added with PyBOP (143 mg, 1.1 eq, 274 µmol). The reaction mixture was cooled to 0 °C, followed by the addition of DIPEA (129 mg, 174 µL, 4 eq, 997 µmol). The resulting clear solution was stirred overnight at room temperature. The reaction mixture was directly subjected to purification by acidic preparative MPLC (Luna 40-80). The product fractions were combined, lyophilized, and subsequently co-evaporated with DCM to afford an orange oil.

Yield: 98 mg, 54%. The product was obtained as a mixture of diastereomer.

¹H NMR (400 MHz, CDCl³) δ 8.22 (s, 1H), 7.25-7.17 (m, 1H), 7.15-6.99 (m, 1H), 6.78 (d, *J* = 8.6 Hz, 1H), 6.65-6.57 (m, 1H), 6.38-6.06 (m, 1H), 6.06-5.91 (m, 1H), 5.41-5.33 (m, 1H), 5.29-5.23 (m, 1H), 5.12-4.81 (m, 2H), 4.75-4.59 (m, 3H), 4.23-4.15 (m, 2H), 3.82 (s, 3H), 2.97-2.79 (m, 6H), 1.91-1.80 (m, 4H), 1.54-1.37 (m, 14H), 1.33-1.22 (m, 4H), 0.90-0.72 (m, 16H). m/z 731.6 [M+H]⁺.

### Step 2:

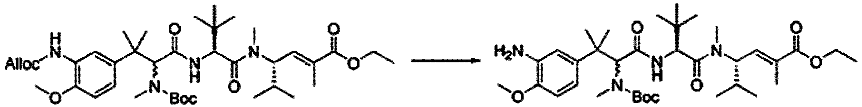

A solution of ethyl (9S,12S,E)-6-(2-(3-(((allyloxy)carbonyl)amino)-4-methoxyphenyl)propan-2-yl)-9-(tert-butyl)-12-isopropyl-2,2,5,11,14-pentamethyl-4,7,10-trioxo-3-oxa-5,8,11-triazapentadec-13-en-15-oate (90.5 mg, 1 eq, 124 µmol) in tetrahydrofuran (6.0 mL) was bubbled with nitrogen. Pd(PPh₃)₄ (7.1 mg, 0.05 eq, 6.19 µmol) was added, followed by the addition of tri-n-butyltin hydride (72.1 mg, 66.1 µL, 2 eq, 248 µmol). The mixture was stirred under nitrogen atmosphere at room temperature for 2 hours. The solvent was removed under reduced pressure, and the crude material was purified by flash column chromatography (12 g SiO₂, 0-10% methanol in dichloromethane). The fractions were combined and concentrated to afford 79 mg of an orange oil. Further purification by flash column chromatography (4 g SiO₂, 0-40% ethyl acetate in heptane) gave the product as a white solid.

Yield: 56 mg, 70%.

¹H NMR (400 MHz, CDCl₃) δ 7.01-6.55 (m, 4H), 6.28-5.98 (m, 1H), 5.11-5.00 (m, 1H), 4.60 (dd, *J* = 39.1, 9.2 Hz, 1H), 4.24-4.14 (m, 2H), 3.86-3.71 (m, 5H), 2.98-2.81 (m, 6H), 1.86 (d, *J* = 10.1 Hz, 4H), 1.51-1.18 (m, 19H), 0.91-0.68 (m, 16H). m/z 647.2 [M+H]⁺.

### Step 3:

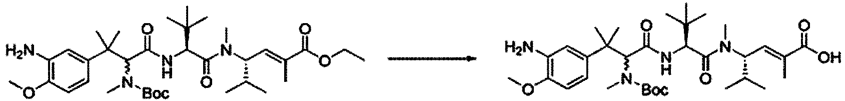

A solution of ethyl (9S,12S,E)-6-(2-(3-amino-4-methoxyphenyl)propan-2-yl)-9-(tert-butyl)-12-isopropyl-2,2,5,11,14-pentamethyl-4,7,10-trioxo-3-oxa-5,8,11-triazapentadec-13-en-15-oate (50.5 mg, 1 eq, 78.1 µmol) in methanol (2.0 mL) was added with a solution of lithium hydroxide monohydrate (16.4 mg, 5 eq, 390 µmol) in water (1.0 mL). The solution turned yellow. The reaction mixture was stirred at room temperature overnight. The reaction mixture was neutralized with acetic acid and extracted with ethyl acetate (3x). The organic layers were washed with brine, dried over sodium sulfate, concentrated, and co-evaporated with dichloromethane (2x) to afford a beige solid.

Yield: 47 mg, 97%.

m/z 619.2 [M+H]⁺.

### Step 4:

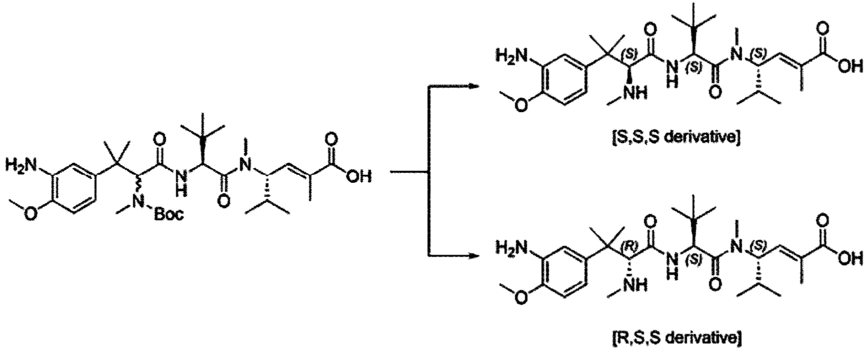

A solution of (9S,12S,E)-6-(2-(3-amino-4-methoxyphenyl)propan-2-yl)-9-(tert-butyl)-12-isopropyl-2,2,5,11,14-pentamethyl-4,7,10-trioxo-3-oxa-5,8,11-triazapentadec-13-en-15-oic acid (46 mg, 1 eq, 74 µmol) in dichloromethane (1.0 mL) was added with 10% TFA in dichloromethane (1.0 mL), and the reaction mixture was stirred at room temperature for 1 hour. The mixture was cooled to 0 °C and TFA (740 mg, 500 µL, 87 eq, 6.49 mmol) was added dropwise. The reaction mixture was stirred at room temperature for 1 hour. The solvent was removed under reduced pressure, and the residue was purified by acidic preparative MPLC (Luna 5-40). The product fractions were combined and lyophilized.

Yield: 19 mg, 50%.

The product was obtained as a mixture of diastereomers. The diastereomers were separated by RP-HPLC to give the S,S,S isomer (5 mg) and the R,S,S isomer (7 mg).

¹H NMR (400 MHz, DMSO-d₆) δ 7.76 (s, 1H), 6.76-6.54 (m, 4H), 5.31-4.01 (m, 4H), 3.73 (d, *J* = 2.9 Hz, 3H), 3.30 (s, 2H), 3.16-2.89 (m, 4H), 2.12-1.89 (m, 4H), 1.78 (d, *J* = 1.4 Hz, 3H), 1.28-1.10 (m, 6H), 0.90 (d, *J* = 24.6 Hz, 9H), 0.83-0.67 (m, 6H). m/z 519.4 [M+H]⁺.

### [Example 2]

### Synthesis of Compound of Chemical Formula 4 and 5

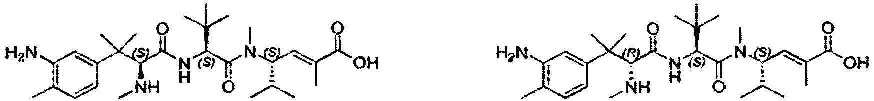

### 2-1. Synthesis of A-fragment

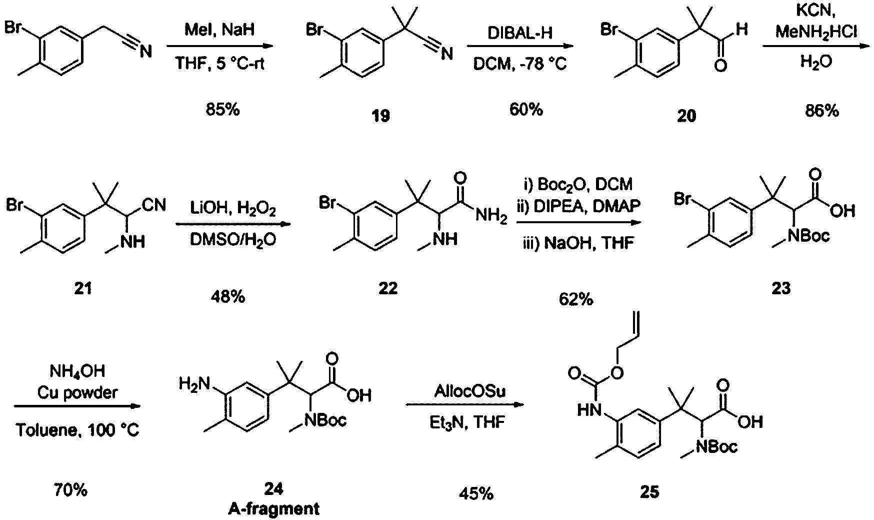

### Step 1:

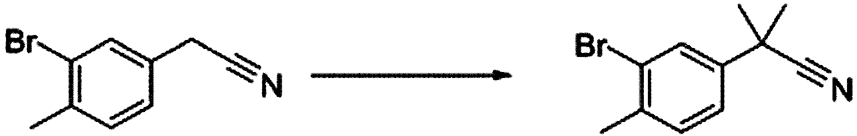

A solution of 2-(3-bromo-4-methylphenyl)acetonitrile (4.64 g, 1 eq, 22.1 mmol) in THF(dry)(30 mL) was added with methyl iodide (7.53 g, 3.30 mL, 2.4 eq, 53.1 mmol). The reaction mixture was cooled to 4 °C and sodium hydride (60% dispersion in mineral oil) (2.12 g, 60% Wt, 2.4 eq, 53.1 mmol) was added portionwise. After the completion of the addition, the reaction mixture was warmed to room temperature and stirred overnight. Methyl iodide (628 mg, 277 µL, 0.2 eq, 4.42 mmol) and sodium hydride (60% dispersion in mineral oil) (177 mg, 60% Wt, 0.2 eq, 4.42 mmol) were further added, and the reaction mixture was stirred at room temperature for 1 hour. The reaction mixture was poured into ice-water (~100 mL) and extracted with EtOAc(3x). The combined organic layers were washed with brine, dried over Na₂SO₄, and concentrated under reduced pressure to give a brown oil (6.47 g). The crude product was purified by flash column chromatography (120 g SiO₂, 0-20% EtOAc in heptane), and the product fractions were concentrated under reduced pressure to afford a colorless oil.

Yield: 4.47 g, 85%.

¹H NMR (400 MHz, CDCl³) δ 7.60 (d, *J* = 2.6 Hz, 1H), 7.37 (dd, *J* = 8.6, 2.5 Hz, 1H), 6.89 (d, *J* = 8.7 Hz, 1H), 2.34 (s, 3H), 1.71 (s, 6H). m/z 238.2/240.2 [M+H]⁺, Br isotope pattern.

### Step 2:

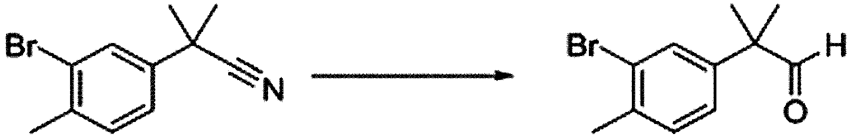

A solution of 2-(3-bromo-4-methylphenyl)-2-methylpropanenitrile (4.38 g, 1 eq, 18.4 mmol) in DCM(dry)(40 mL) was cooled to -78 °C. Diisobutylaluminum hydride (1 M in hexane) (3.40 g, 23.9 mL, 1.0 molar, 1.3 eq, 23.9 mmol) was added dropwise to the solution within 15 minutes. The reaction mixture was stirred at -78 °C for 1.5 hours. Diisobutylaluminum hydride (1 M in hexane) (655 mg, 4.60 mL, 1.0 molar, 0.25 eq, 4.60 mmol) was further added dropwise, and the reaction mixture was stirred at -78 °C for 30 minutes. The reaction mixture was warmed to 0 °C, quenched slowly with 2 M hydrochloric acid (60 mL), stirred at 0 °C for 30 minutes, and then stirred at room temperature for 30 minutes. The mixture was diluted with water and extracted with DCM (3x). The combined organic layers were washed with brine, dried over Na₂SO₄, and concentrated under reduced pressure to give a solid (4.39 g). The crude product was suspended in DCM and filtered. The filtrate was purified by column chromatography (120 g SiO₂, 0-20% EtOAc in heptane). The product fractions were concentrated under reduced pressure to afford a light-yellow oil (2.68 g, 60%).

¹H NMR (400 MHz, CDCl₃) δ 9.42 (s, 1H), 7.41 (d, *J* = 2.4 Hz, 1H), 7.13 (dd, *J* = 8.6, 2.4 Hz, 1H), 6.85 (d, *J* = 8.6 Hz, 1H), 2.33 (s, 3H), 1.43 (s, 6H). m/z 241.2/243.2 [M+H]⁺, Br isotope pattern.

### Step 3:

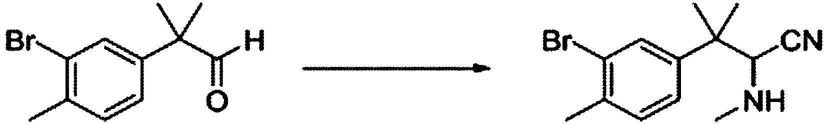

In a 100 mL round-bottom flask, potassium cyanide (0.56 g, 1 eq, 8.6 mmol) and water (13 mL) were charged. The solution was cooled to 0 °C, and methylamine hydrochloride (0.58 g, 1 eq, 8.6 mmol) was added. Thereafter, a solution of 2-(3-bromo-4-methylphenyl)-2-methylpropanal (2.25 g, 1 eq, 8.6 mmol) in methanol (13 mL) was added. The resulting white suspension was stirred at room temperature overnight. The reaction mixture was diluted with H₂O (40 mL) and extracted with CH₂Cl₂ (3 × 50 mL). The combined organic layers were washed with brine (20 mL), dried over Na₂SO₄, and concentrated under vacuum to afford 3-(3-bromo-4-methylphenyl)-3-methyl-2-(methylamino)butanenitrile as a colorless oil.

Yield: 2.2 g, 86%.

¹H NMR (400 MHz, CDCl³) δ 7.52 (d, *J* = 2.4 Hz, 1H), 7.31 (dd, *J* = 8.7, 2.4 Hz, 1H), 6.84 (d, *J* = 8.7 Hz, 1H), 3.43-3.31 (m, 1H), 2.46 (s, 3H), 2.33 (s, 3H), 1.50 (d, *J* = 11.5 Hz, 6H). m/z 281.0/283.0 [M+H]⁺, Br isotope pattern.

### Step 4:

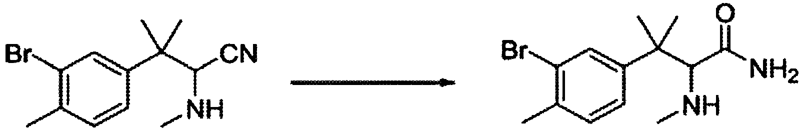

A solution of 3-(3-bromo-4-methylphenyl)-3-methyl-2-(methylamino)butanenitrile (2.39 g, 1 eq, 8.51 mmol) in DMSO (45 mL) was added with an aqueous solution of lithium hydroxide monohydrate (1 M, 1.71 g, 40.9 mL, 4.8 eq, 40.9 mmol) and hydrogen peroxide (4.63 g, 4.17 mL, 30% Wt, 4.8 eq, 40.9 mmol). The reaction mixture was stirred at room temperature for 2 hours. The reaction mixture was diluted with water (385 mL) and extracted with ethyl acetate (2 × 385 mL). The combined organic layers were washed with water and brine, dried over sodium sulfate, and concentrated under reduced pressure to afford a colorless viscous solid (2.5 g). The crude product was purified by flash column chromatography (80 g SiO₂, 0-5% methanol in dichloromethane) to afford the desired product as a white foam.

Yield: 1.23 g, 48%.

¹H NMR (400 MHz, DMSO) δ 7.46 (d, *J* = 2.4 Hz, 1H), 7.33-7.21 (m, 2H), 7.02-6.95 (m, 2H), 2.97-2.87 (m, 1H), 2.30 (s, 3H), 2.07 (s, 3H), 1.44 (bs, 1H), 1.23 (d, *J* = 7.8 Hz, 6H). m/z 299.2/301.2 [M+H]⁺, Br isotope pattern.

### Step 5:

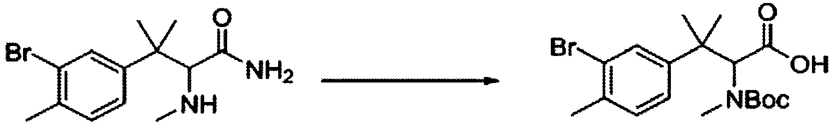

A solution of 3-(3-bromo-4-methylphenyl)-3-methyl-2-(methylamino)butanamide (1.24 g, 1 eq, 4.16 mmol) in dichloromethane (15 mL) was prepared. Boc₂O (3.17 g, 3.5 eq, 14.5 mmol) was then added. After several minutes, a clear and colorless solution was obtained. The reaction mixture was stirred at room temperature for 3 days. DIPEA (591 mg, 787 µL, 1.1 eq, 4.57 mmol) and DMAP (50.8 mg, 0.1 eq, 416 µmol) were added, and the reaction mixture was stirred at room temperature for 2 hours. Water (45 mL) was added, and the mixture was extracted with heptane (3 × 50 mL). The organic layer was washed with water (25 mL) and brine (25 mL), dried over sodium sulfate, and concentrated to dryness, affording a yellow oil (2.8 g). The yellow oil was dissolved in THF (6.5 mL), and sodium hydroxide 5M (831 mg, 4.16 mL, 5.0 molar, 5 eq, 20.8 mmol) was added. The reaction mixture was stirred at room temperature for 2 hours, followed by addition of water(25 mL). The mixture was filtered, and the residue was washed with 0.5 M NaOH (25 mL). The THF was removed under reduced pressure. The aqueous solution was washed with heptane, neutralized with solid citric acid, and extracted with ethyl acetate (3×). The combined organic layers were washed with brine, dried over sodium sulfate, and concentrated under reduced pressure to give a colorless oil (1.35 g). The crude product was purified by column chromatography (40 g SiO₂, 0-50% ethyl acetate in heptane) to afford the desired product as a white foam.

Yield: 1.04 g, 62%.

¹H NMR (400 MHz, CDCl₃) δ 7.55-7.46 (m, 1H), 7.33-7.22 (m, 1H), 6.78 (d, *J* = 8.7 Hz, 1H), 5.03-4.80 (m, 1H), 2.82-2.60 (m, 3H), 2.33 (s, 3H), 1.53-1.35 (m, 15H). m/z 398.2/400.2 [M-H]⁻, Br isotope pattern.

### Step 6:

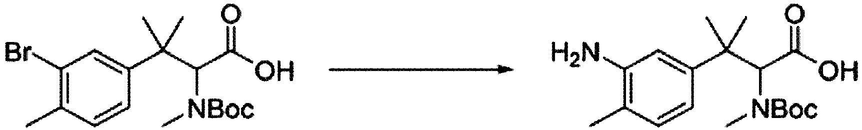

A mixture of 3-(3-bromo-4-methylphenyl)-2-((tert-butoxycarbonyl)(methyl)amino)-3-methylbutanoic acid (354 mg, 1 eq, 841 µmol) and toluene (5.0 mL) was placed in a 20 mL microwave vial. To this mixture, ammonium hydroxide solution, 32% (9.21 g, 10.2 mL, 32% Wt, 100 eq, 84.1 mmol) was added, followed by copper powder (160 mg, 3 eq, 2.52 mmol). The vial was sealed and stirred at 100 °C for 6 hours. The reaction mixture was filtered, and the filtrate was evaporated to dryness. The residue was co-evaporated with ethanol (3×) to remove residual water. The crude material was coated onto hydromatrix and purified by flash column chromatography (12 g SiO₂, 0-10% methanol in dichloromethane). The combined product fractions were concentrated under reduced pressure to afford 232 mg of the product as a green oil/foam. The product was used directly in the next step without further purification.

Yield: 70%.

¹H NMR (400 MHz, CDCl₃) δ 7.18-5.90 (m, 3H), 5.15-4.65 (m, 1H), 2.89-2.39 (m, 4H), 2.33-2.28 (m, 3H), 1.90-1.05 (m, 17H). m/z 673.2 [2M+H]⁺.

### Step 7:

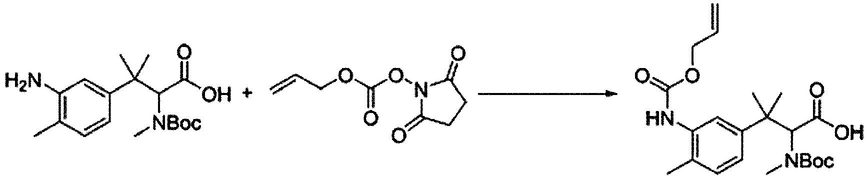

A solution of 3-(3-amino-4-methylphenyl)-2-((tert-butoxycarbonyl)(methyl)amino)-3-methylbutanoic acid (196 mg, 1 eq, 582 µmol) in tetrahydrofuran (1.5 mL) was treated with Alloc-OSu (232 mg, 2 eq, 1.16 mmol), followed by triethylamine (177 mg, 243 µL, 3 eq, 1.75 mmol). The reaction mixture was stirred at room temperature overnight. The solvent was removed under reduced pressure and the residue was purified by flash column chromatography (12 g SiO₂, 0-10% methanol in dichloromethane). The product fractions were concentrated to afford 110 mg (45%) of a light brown oil. The NMR spectrum indicated the rotamers.

¹H NMR (400 MHz, CDCl₃) δ 8.18 (s, 1H), 7.21 (s, 1H), 7.08-6.95 (m, 1H), 6.75 (d, *J* = 8.6 Hz, 1H), 6.02-5.90 (m, 1H), 5.39-5.31 (m, 1H), 5.25-5.20 (m, 1H), 4.91 (s, 1H), 4.68-4.62 (m, 2H), 2.75 (s, 1H), 2.63 (s, 2H), 2.31 (s, 3H), 1.52-1.38 (m, 16H). m/z 443.2 [M+Na]⁺.

### 2-2. Synthesis of Compound of Chemical Formula 4 and 5

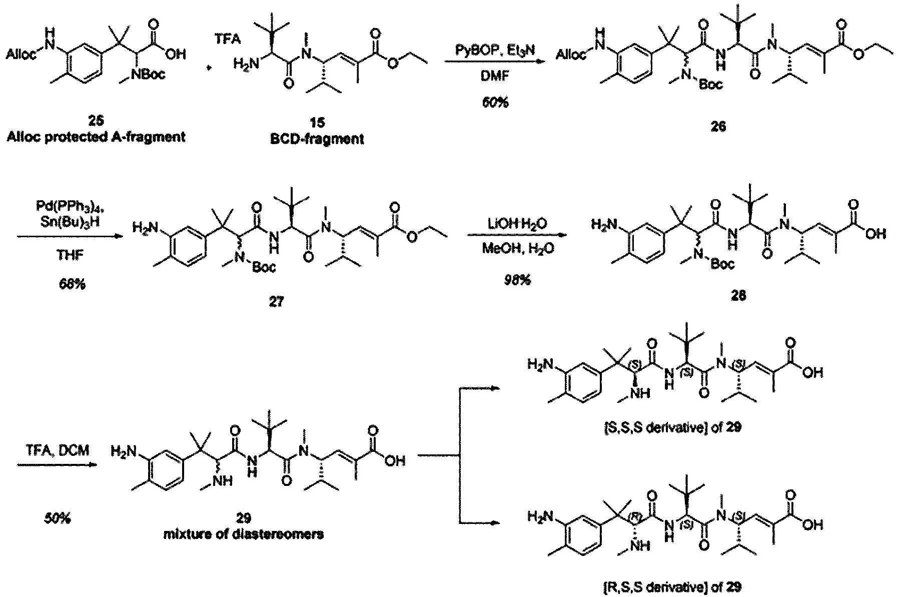

### Step 1:

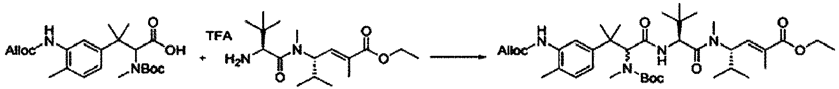

A solution of 3-(3-(((allyloxy)carbonyl)amino)-4-methylphenyl)-2-((tert-butoxycarbonyl)(methyl)amino)-3-methylbutanoic acid [A fragment] (123 mg, 85% Wt, 1 eq, 249 µmol) in N,N-dimethylformamide (1.8 mL) was treated with ethyl (S,E)-4-((S)-2-amino-N,3,3-trimethylbutanamido)-2,5-dimethylhex-2-enoate 2,2,2-trifluoroacetate [BCD fragment] (142 mg, 75% Wt, 1 eq, 249 µmol), followed by addition of PyBOP (143 mg, 1.1 eq, 274 µmol). The reaction mixture was cooled to 0 °C, and DIPEA (129 mg, 174 µL, 4 eq, 997 µmol) was added. The resulting clear solution was stirred at room temperature overnight. The reaction mixture was directly subjected to purification by acidic preparative MPLC (Luna 40-80). The product fractions were combined, lyophilized, and co-evaporated with DCM to afford an orange oil.

Yield: 108 mg, 60%. The product was obtained as a mixture of diastereomers.

¹H NMR (400 MHz, CDCl₃) δ 8.20 (s, 1H), 7.23-7.12 (m, 1H), 7.12-6.96 (m, 1H), 6.75 (d, *J* = 8.6 Hz, 1H), 6.63-6.54 (m, 1H), 6.35-6.03 (m, 1H), 6.03-5.88 (m, 1H), 5.38-5.30 (m, 1H), 5.25-5.20 (m, 1H), 5.10-4.78 (m, 2H), 4.72-4.55 (m, 3H), 4.20-4.12 (m, 2H), 2.95-2.75 (m, 6H), 2.31 (s, 3H), 1.89-1.78 (m, 4H), 1.52-1.32 (m, 14H), 1.31-1.20 (m, 4H), 0.89-0.70 (m, 16H). m/z 715.6 [M+H]⁺.

### Step 2:

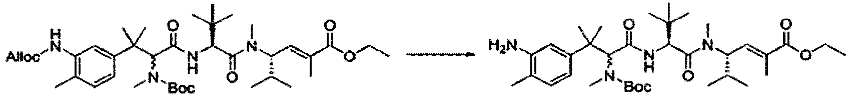

A solution of ethyl (9S,12S,E)-6-(2-(3-(((allyloxy)carbonyl)amino)-4-methylphenyl)propan-2-yl)-9-(tert-butyl)-12-isopropyl-2,2,5,11,14-pentamethyl-4,7,10-trioxo-3-oxa-5,8,11-triazapentadec-13-en-15-oate (88.6 mg, 1 eq, 124 µmol) in tetrahydrofuran (6.0 mL) was bubbled with nitrogen. Pd(PPh₃)₄ (7.1 mg, 0.05 eq, 6.19 µmol) was added, and then tri-n-butyltin hydride (72.1 mg, 66.1 µL, 2 eq, 248 µmol) was added. The resulting mixture was stirred at room temperature under a nitrogen atmosphere for 2 hours. And then the solvent was removed under reduced pressure, and the crude materials was purified by flash column chromatography (12 g SiO₂, 0-10% methanol in dichloromethane). The fractions were combined and then concentrated to afford 79 mg of an orange oil. Further purification by flash column chromatography (4 g SiO₂, 0-40% ethyl acetate in heptane) gave the product as a white solid.

Yield: 53 mg, 68%.

¹H NMR (400 MHz, CDCl₃) δ 6.98-6.52 (m, 4H), 6.25-5.95 (m, 1H), 5.06-4.95 (m, 1H), 4.55 (dd, *J* = 39.1, 9.2 Hz, 1H), 4.20-4.10 (m, 2H), 3.83-3.68 (m, 2H), 2.95-2.78 (m, 6H), 2.22 (s, 3H), 1.86 (d, *J* = 10.1 Hz, 4H), 1.51-1.18 (m, 19H), 0.91-0.68 (m, 16H). m/z 631.2 [M+H]⁺.

### Step 3:

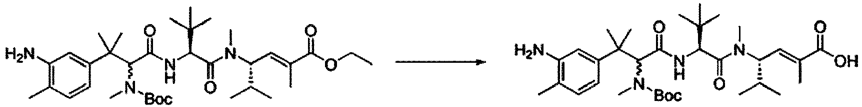

A solution of ethyl (9S,12S,E)-6-(2-(3-amino-4-methylphenyl)propan-2-yl)-9-(tert-butyl)-12-isopropyl-2,2,5,11,14-pentamethyl-4,7,10-trioxo-3-oxa-5,8,11-triazapentadec-13-en-15-oate (49 mg, 1 eq, 78.1 µmol) in methanol (2.0 mL) was treated with a solution of lithium hydroxide monohydrate (16.4 mg, 5 eq, 390 µmol) in water (1.0 mL). The solution turned yellow. The reaction mixture was stirred at room temperature overnight. The reaction mixture was neutralized with acetic acid and extracted with ethyl acetate (3x). The organic layers were washed with brine, dried over sodium sulfate, concentrated, and co-evaporated with dichloromethane (2x) to afford a beige solid.
Yield: 46 mg, 98%
m/z 603.2 [M+H]⁺

### Step 4:

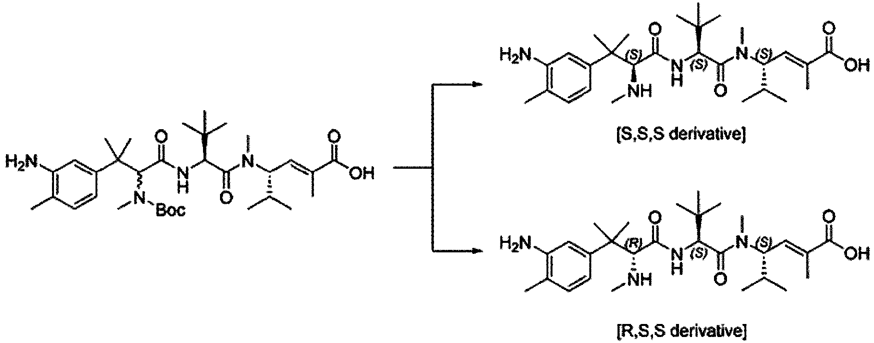

A solution of (9S,12S,E)-6-(2-(3-amino-4-methylphenyl)propan-2-yl)-9-(tert-butyl)-12-isopropyl-2,2,5,11,14-pentamethyl-4,7,10-trioxo-3-oxa-5,8,11-triazapentadec-13-en-15-oic acid (46 mg, 1 eq, 74 µmol) in dichloromethane (1.0 mL) was treated with 10% TFA in dichloromethane (1.0 mL), and the reaction mixture was stirred at room temperature for 1 hour. The mixture was cooled to 0 °C, and TFA (740 mg, 500 µL, 87 eq, 6.49 mmol) was added dropwise. The reaction mixture was stirred at room temperature for 1 hour. The solvent was removed under reduced pressure, and the residue was purified by acidic preparative MPLC (Luna 5-40). The product fractions were combined and lyophilized.

Yield: 19 mg, 50%.

The product was obtained as a mixture of diastereomers. The diastereomers were separated by RP-HPLC to afford the S,S,S isomer (5 mg) and the R,S,S isomer (7 mg).
¹H NMR (400 MHz, DMSO-d₆) δ 7.72 (s, 1H), 6.73-6.51 (m, 4H), 5.30-4.00 (m, 4H), 3.28 (s, 2H), 3.14-2.86 (m, 4H), 2.18 (d, *J* = 2.9 Hz, 3H), 2.10-1.84 (m, 4H), 1.74 (d, *J* = 1.4 Hz, 3H), 1.24-1.08 (m, 6H), 0.89 (d, *J* = 24.6 Hz, 9H), 0.82-0.65 (m, 6H). m/z 503.4 [M+H]⁺

### [Example 3]

### Synthesis of Compound of Chemical Formula 6 and 7

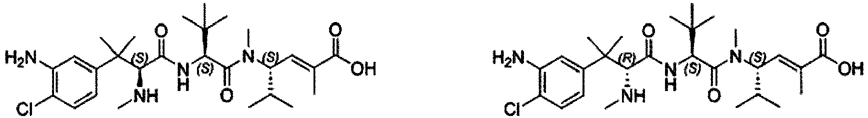

### 3-1. Synthesis of A fragment

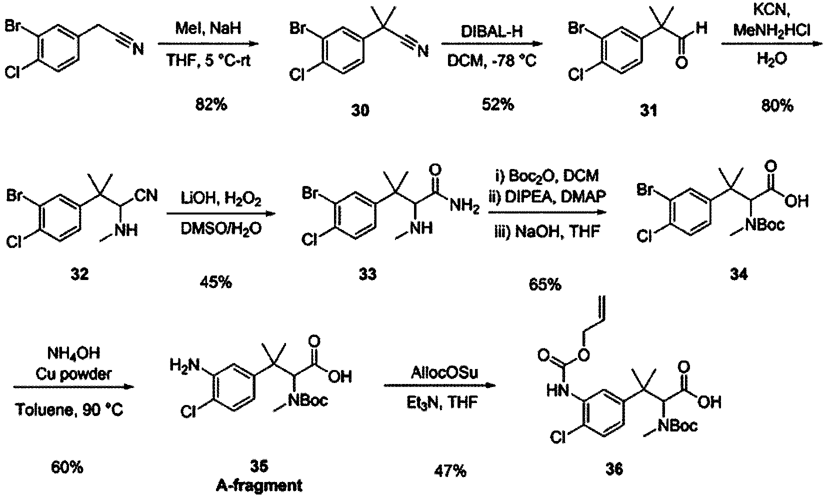

### Step 1:

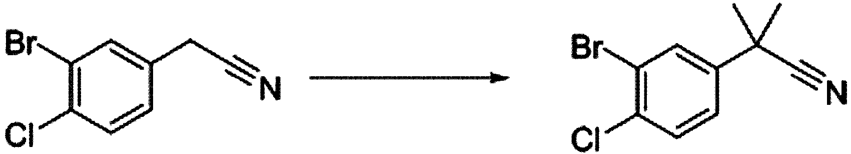

A solution of 2-(3-bromo-4-chlorophenyl)acetonitrile (5.00 g, 1 eq, 22.1 mmol) in THF(dry)(30 mL) was treated with methyl iodide (7.53 g, 3.30 mL, 2.4 eq, 53.1 mmol). The reaction mixture was cooled to 4 °C, and sodium hydride (60% dispersion in mineral oil)(2.12 g, 60% Wt, 2.4 eq, 53.1 mmol) was added portionwise. After completion of the addition, the reaction mixture was warmed to room temperature and stirred overnight. Methyl iodide (628 mg, 277 µL, 0.2 eq, 4.42 mmol) and sodium hydride (60% dispersion in mineral oil)(177 mg, 60% Wt, 0.2 eq, 4.42 mmol) were further added, and the reaction mixture was stirred at room temperature for 1 hour. The reaction mixture was poured into ice-water (~100 mL) and extracted with EtOAc(3×). The combined organic layers were washed with brine, dried over Na₂SO₄, and concentrated under reduced pressure to give a brown oil (6.47 g). The brown oil was purified by flash column chromatography (120 g SiO₂, 0-20% EtOAc in heptane), and the product fractions were concentrated under reduced pressure to afford a colorless oil.

Yield: 4.7 g, 82%.

¹H NMR (400 MHz, CDCl₃) δ 7.52 (d, *J* = 2.6 Hz, 1H), 7.34 (dd, *J* = 8.6, 2.5 Hz, 1H), 7.15 (d, *J* = 8.7 Hz, 1H), 1.70 (s, 6H). m/z 257.2/259.2 [M+H]⁺, Br isotope pattern.

### Step 2:

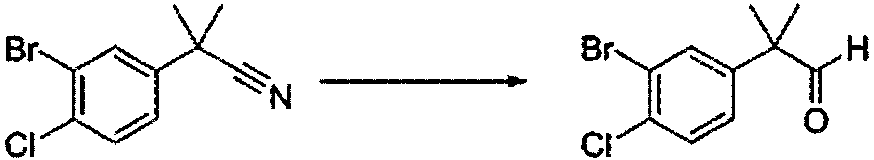

A solution of 2-(3-bromo-4-chlorophenyl)-2-methylpropanenitrile (4.76 g, 1 eq, 18.4 mmol) in DCM(dry)(40 mL) was cooled to -78 °C. Diisobutylaluminum hydride (1 M in hexane)(3.40 g, 23.9 mL, 1.0 molar, 1.3 eq, 23.9 mmol) was added dropwise to the solution within 15 minutes. The reaction mixture was stirred at -78 °C for 1.5 hours. Additional diisobutylaluminum hydride (1 M in hexane) (655 mg, 4.60 mL, 1.0 molar, 0.25 eq, 4.60 mmol) was added dropwise, and the mixture was stirred at -78 °C for 30 minutes. The reaction mixture was warmed to 0 °C, quenched slowly with 2 M HCI (60 mL), and stirred for 30 minutes at 0 °C, followed by stirring for 30 minutes at room temperature. The mixture was diluted with water and extracted with DCM (3×). The combined organic layers were washed with brine, dried over Na₂SO₄, and concentrated under reduced pressure to afford a solid (4.39 g). The crude product was suspended in DCM and filtered. The filtrate was purified by column chromatography (120 g SiO₂, 0-20% EtOAc in heptane). The product fractions were concentrated under reduced pressure to give a light-yellow oil (2.49 g, 52%).

¹H NMR (400 MHz, CDCl₃) δ 9.45 (s, 1H), 7.51 (d, *J* = 2.3 Hz, 1H), 7.35 (dd, *J* = 8.5, 2.3 Hz, 1H), 7.18 (d, *J* = 8.5 Hz, 1H), 1.45 (s, 6H). m/z 260.2/262.2 [M+H]⁺, Br isotope pattern.

### Step 3:

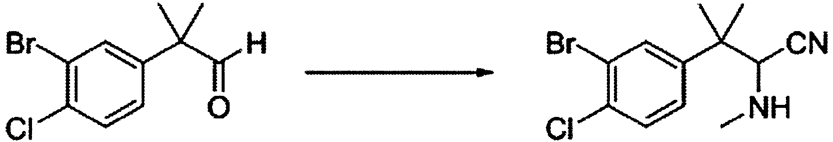

A 100 mL round-bottom flask was charged with potassium cyanide (0.56 g, 1 eq, 8.6 mmol) and water (13 mL). The solution was cooled to 0 °C, and methylamine hydrochloride (0.58 g, 1 eq, 8.6 mmol) was added. Subsequently, a solution of 2-(3-bromo-4-chlorophenyl)-2-methylpropanal (2.25 g, 1 eq, 8.6 mmol) in methanol (13 mL) was added. The resulting white suspension was stirred at room temperature overnight. The reaction mixture was diluted with H₂O (40 mL) and extracted with CH₂Cl₂ (3 × 50 mL). The combined organic layers were washed with brine (20 mL), dried over Na₂SO₄, and concentrated under vacuum to afford 3-(3-bromo-4-chlorophenyl)-3-methyl-2-(methylamino)butanenitrile as a colorless oil.

Yield: 2.08 g, 80%.

¹H NMR (400 MHz, CDCl₃) δ 7.58 (d, *J* = 2.4 Hz, 1H), 7.33 (dd, *J* = 8.7, 2.4 Hz, 1H), 7.09 (d, *J* = 8.7 Hz, 1H), 3.45-3.35 (m, 1H), 2.48 (s, 3H), 1.52 (d, *J* = 11.5 Hz, 6H). m/z 300.4/302.4 [M+H]⁺, Br isotope pattern.

### Step 4:

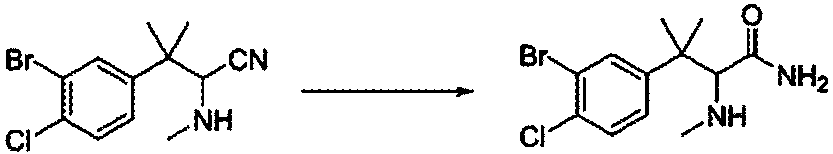

A solution of 3-(3-bromo-4-chlorophenyl)-3-methyl-2-(methylamino)butanenitrile (2.57 g, 1 eq, 8.51 mmol) in DMSO (45 mL) was treated with a 1 M lithium hydroxide monohydrate (1.71 g, 40.9 mL, 1.0 molar, 4.8 eq, 40.9 mmol) in water and hydrogen peroxide (4.63 g, 4.17 mL, 30% w/w, 4.8 eq, 40.9 mmol). The reaction mixture was stirred at room temperature for 2 hours. The reaction mixture was diluted with water (385 mL) and extracted with ethyl acetate (2 × 385 mL). The organic layers were washed with water and brine, dried over sodium sulfate, and concentrated under reduced pressure to afford a colorless sticky solid (2.5 g). The solid was purified by flash column chromatography (80 g SiO₂, 0-5% methanol in dichloromethane) to give the product as a white foam.

Yield: 1.23 g, 45%.

1H NMR (400 MHz, DMSO) δ 7.52 (d, *J* = 2.4 Hz, 1H), 7.38-7.28 (m, 2H), 7.18-7.13 (m, 2H), 3.00-2.95 (m, 1H), 2.10 (s, 3H), 1.48 (bs, 1H), 1.25 (d, *J* = 7.8 Hz, 6H). m/z 318.4/320.4 [M+H]⁺, Br isotope pattern.

### Step 5:

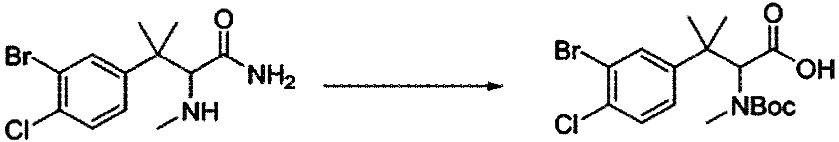

dichloromethane (15 mL) was added to 3-(3-bromo-4-chlorophenyl)-3-methyl-2-(methylamino)butanamide (1.33 g, 1 eq, 4.16 mmol). And then Boc₂O (3.17 g, 3.5 eq, 14.5 mmol) was added. After several minutes, a clear colorless solution was obtained. The reaction mixture was stirred at room temperature for 3 days. DIPEA (591 mg, 787 µL, 1.1 eq, 4.57 mmol) and DMAP (50.8 mg, 0.1 eq, 416 µmol) were then added, and the mixture was stirred at room temperature for 2 hours. Water (45 mL) was added, and the mixture was extracted with heptane (3 × 50 mL). The combined organic layers were washed with water (25 mL) and brine (25 mL), dried over sodium sulfate, and concentrated under reduced pressure to afford a yellow oil (2.8 g), which was dissolved in THF (6.5 mL). Sodium hydroxide 5 M (831 mg, 4.16 mL, 5 eq, 20.8 mmol) was added to this solution, and the mixture was stirred at room temperature for 2 hours. Water (25 mL) was then added, and the mixture was filtered. The residue was washed with 0.5 M NaOH (25 mL). The THF was removed under reduced pressure. The aqueous solution was washed with heptane, neutralized with solid citric acid, and extracted with ethyl acetate (3 ×). The combined organic layers were washed with brine, dried over sodium sulfate, and concentrated under reduced pressure to give a colorless oil (1.37 g). Purification by column chromatography (40 g SiO₂, 0-50% ethyl acetate in heptane) and subsequent concentration of the product fractions under reduced pressure afforded a white foam.

Yield: 1.13 g, 65%.

¹H NMR (400 MHz, CDCl₃) δ 7.58-7.48 (m, 1H), 7.39-7.32 (m, 1H), 7.18 (d, *J* = 8.7 Hz, 1H), 5.13-4.90 (m, 1H), 2.92-2.70 (m, 3H), 1.58-1.45 (m, 15H). m/z 419.2/421.2 [M-H]⁻, Br isotope pattern.

### Step 6:

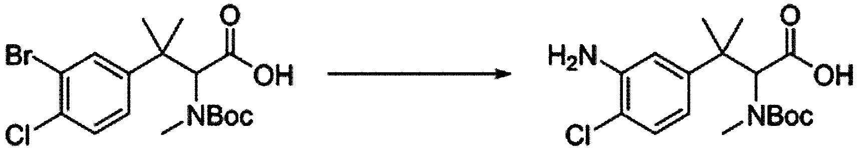

A mixture of 3-(3-bromo-4-chlorophenyl)-2-((tert-butoxycarbonyl)(methyl)amino)-3-methylbutanoic acid (354 mg, 1 eq, 841 µmol) and toluene (5.0 mL) was placed in a 20 mL microwave vial. Ammonium hydroxide solution, 32% (9.21 g, 10.2 mL, 32 wt%, 100 eq, 84.1 mmol), was added, followed by copper powder (160 mg, 3 eq, 2.52 mmol). The vial was sealed and the mixture was stirred at 100 °C for 6 h. The reaction mixture was filtered, and the filtrate was evaporated to dryness. The residue was co-evaporated with ethanol (3×) to remove water. The crude product was coated onto Hydromatrix and purified by flash column chromatography (12 g SiO₂, 0-10% MeOH in dichloromethane). The product fractions were concentrated under reduced pressure to afford the compound (232 mg) as a green oil/foam. The product was directly subjected to the next step.
Yield: 60%.
¹H NMR (400 MHz, CDCl₃) δ 7.28-6.90 (m, 2H), 6.85-6.30 (m, 1H), 5.25-4.85 (m, 1H), 2.95-2.59 (m, 4H), 1.92-1.10 (m, 17H). m/z 713.4 [2M+H]⁺

### Step 7:

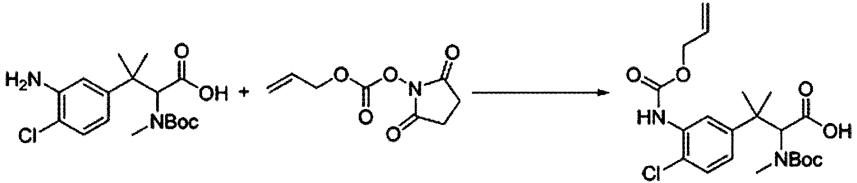

A solution of 3-(3-amino-4-chlorophenyl)-2-((tert-butoxycarbonyl)(methyl)amino)-3-methylbutanoic acid (205 mg, 1 eq, 582 µmol) in tetrahydrofuran (1.5 mL) was treated with Alloc-OSu (232 mg, 2 eq, 1.16 mmol), followed by triethylamine (177 mg, 243 µL, 3 eq, 1.75 mmol). The reaction mixture was stirred at room temperature overnight. The solvent was removed under reduced pressure, and the residue was purified by flash column chromatography (12 g SiO₂, 0-10% MeOH in dichloromethane). The product fractions were concentrated to afford the product as a light-brown oil (120 mg, 47%). The NMR indicated the rotamers.
¹H NMR (400 MHz, CDCl₃) δ 8.28 (s, 1H), 7.31 (s, 1H), 7.25-7.10 (m, 1H), 6.95 (d, *J* = 8.6 Hz, 1H), 6.12-6.05 (m, 1H), 5.45-5.36 (m, 1H), 5.28-5.23 (m, 1H), 4.96 (s, 1H), 4.72-4.68 (m, 2H), 2.85 (s, 1H), 2.73 (s, 2H), 1.55-1.42 (m, 16H). m/z 463.2 [M+Na]⁺

### 3-2. Synthesis of Compound of Chemical Formula 6 and 7.

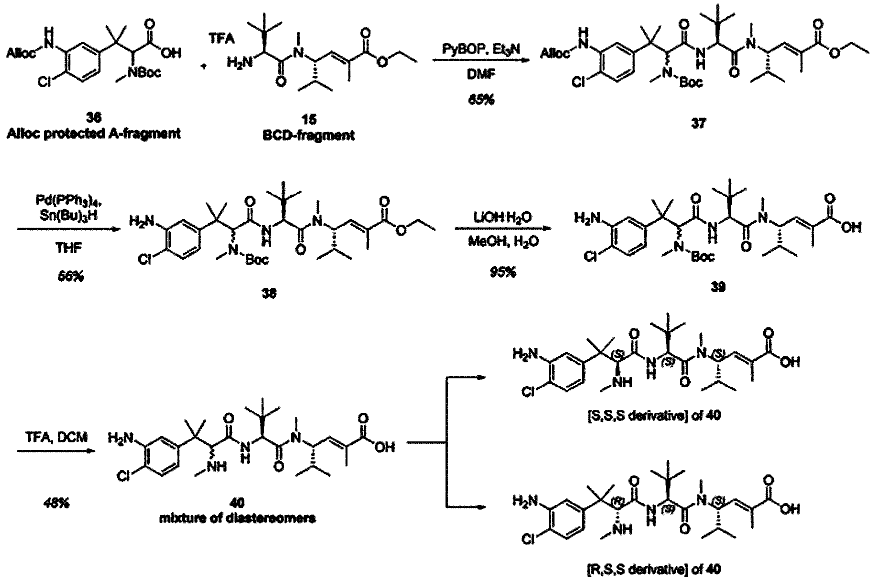

### Step 1:

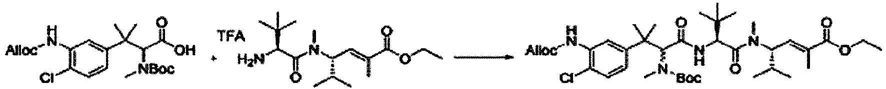

A solution of 3-(3-(((allyloxy)carbonyl)amino)-4-chlorophenyl)-2-((tert-butoxycarbonyl)(methyl)amino)-3-methylbutanoic acid [A Fragment] (125 mg, 88% Wt, 1 eq, 249 µmol) in N,N-dimethylformamide (1.8 mL) was treated with ethyl (S,E)-4-((S)-2-amino-N,3,3-trimethylbutanamido)-2,5-dimethylhex-2-enoate 2,2,2-trifluoroacetate [BCD Fragment] (142 mg, 75% Wt, 1 eq, 249 µmol), followed by addition of PyBOP (143 mg, 1.1 eq, 274 µmol). The reaction mixture was cooled to 0 °C, and DIPEA (129 mg, 174 µL, 4 eq, 997 µmol) was added. The resulting clear solution was stirred overnight at room temperature. The reaction mixture was directly subjected to acidic preparative MPLC (Luna 40-80). The product fractions were combined, lyophilized and subsequently co-evaporated with dichloromethane to afford an orange oil.

Yield: 120 mg, 65%. The product was obtained as a mixture of diastereomers.
¹H NMR (400 MHz, CDCl₃) δ 8.30 (s, 1H), 7.33-7.22 (m, 1H), 7.22-7.06 (m, 1H), 6.85 (d, *J* = 8.6 Hz, 1H), 6.73-6.64 (m, 1H), 6.45-6.13 (m, 1H), 6.13-5.98 (m, 1H), 5.48-5.40 (m, 1H), 5.35-5.30 (m, 1H), 5.20-4.88 (m, 2H), 4.82-4.65 (m, 3H), 4.30-4.22 (m, 2H), 3.05-2.85 (m, 6H), 1.95-1.85 (m, 4H), 1.55-1.38 (m, 14H), 1.35-1.25 (m, 4H), 0.92-0.75 (m, 16H). m/z 735.2 [M+H]⁺

### Step 2:

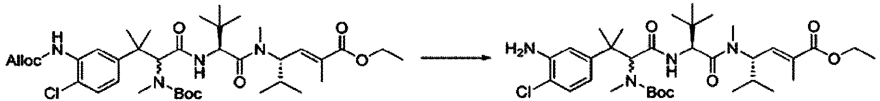

A solution of ethyl (9S,12S,E)-6-(2-(3-(((allyloxy)carbonyl)amino)-4-chlorophenyl)propan-2-yl)-9-(tert-butyl)-12-isopropyl-2,2,5,11,14-pentamethyl-4,7,10-trioxo-3-oxa-5,8,11-triazapentadec-13-en-15-oate (91 mg, 1 eq, 124 µmol) in tetrahydrofuran (6.0 mL) was bubbled with nitrogen. Pd(PPh₃)₄ (7.1 mg, 0.05 eq, 6.19 µmol) was added, followed by tri-n-butyltin hydride (72.1 mg, 66.1 µL, 2 eq, 248 µmol). The mixture was stirred at room temperature under nitrogen atmosphere for 2 h. The solvent was removed under reduced pressure, and the crude material was purified by flash column chromatography (12 g SiO₂, 0-10% methanol in dichloromethane). The fractions were combined, concentrated to afford an orange oil (79 mg). Further purification by flash column chromatography (4 g SiO₂, 0-40% ethyl acetate in heptane) furnished the product as a white solid.
Yield: 53.2 mg (66%).
¹H NMR (400 MHz, CDCl₃) δ 7.08-6.62 (m, 4H), 6.35-6.05 (m, 1H), 5.16-5.05 (m, 1H), 4.65 (dd, *J* = 39.1, 9.2 Hz, 1H), 4.30-4.20 (m, 2H), 3.93-3.78 (m, 2H), 3.05-2.88 (m, 6H), 1.96 (d, *J* = 10.1 Hz, 4H), 1.61-1.28 (m, 19H), 0.95-0.72 (m, 16H). m/z 651.2 [M+H]⁺

### Step 3:

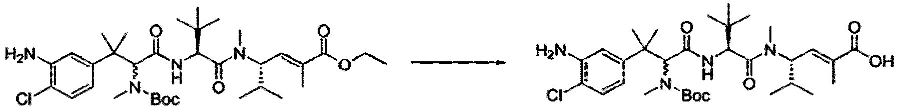

A solution of ethyl (9S,12S,E)-6-(2-(3-amino-4-chlorophenyl)propan-2-yl)-9-(tert-butyl)-12-isopropyl-2,2,5,11,14-pentamethyl-4,7,10-trioxo-3-oxa-5,8,11-triazapentadec-13-en-15-oate (51 mg, 1 eq, 78.1 µmol) in methanol (2.0 mL) was treated with a solution of lithium hydroxide monohydrate (16.4 mg, 5 eq, 390 µmol) in water (1.0 mL). The solution turned yellow, and the reaction mixture was stirred at room temperature overnight. The mixture was neutralized with acetic acid and extracted with ethyl acetate (3×). The organic layers were washed with brine, dried over Na₂SO₄, concentrated, and co-evaporated with dichloromethane (2×) to give a beige solid.
Yield: 46 mg (95%)
m/z: 623.2 [M+H]⁺

### Step 4:

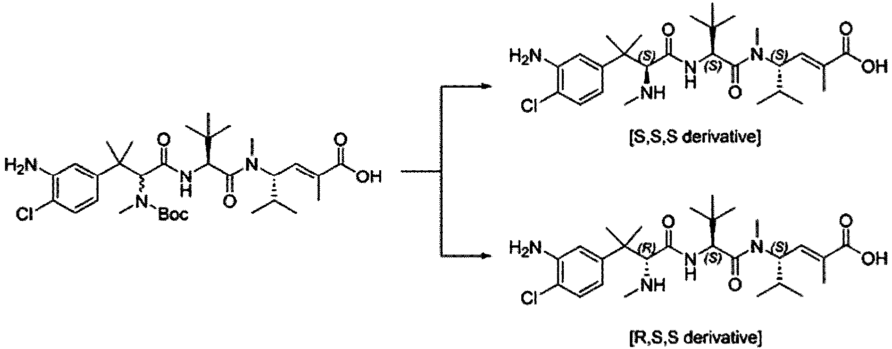

A solution of (9S,12S,E)-6-(2-(3-amino-4-chlorophenyl)propan-2-yl)-9-(tert-butyl)-12-isopropyl-2,2,5,11,14-pentamethyl-4,7,10-trioxo-3-oxa-5,8,11-triazapentadec-13-en-15-oic acid (44 mg, 1 eq, 74 µmol) in dichloromethane (1.0 mL) was treated with 10% TFA in dichloromethane (1.0 mL). The reaction mixture was stirred at room temperature for 1 h, cooled to 0 °C, and TFA (740 mg, 500 µL, 87 eq, 6.49 mmol) was added dropwise. The mixture was stirred at room temperature for an additional 1 h. The solvent was removed under reduced pressure, and the residue was purified by acidic preparative MPLC (Luna 5-40). The product fractions were combined and lyophilized.

Yield: 17.8 mg, 48%.

The product was obtained as a mixture of diastereomers. The diastereomers were separated by RP-HPLC to afforded the S,S,S isomer (5.5 mg) and the R,S,S isomer (7.5 mg).
¹H NMR (400 MHz, DMSO-d₆) δ 7.82 (s, 1H), 6.83-6.61 (m, 4H), 5.40-4.10 (m, 4H), 3.38 (s, 2H), 3.24-2.96 (m, 4H), 2.20-1.94 (m, 4H), 1.84 (d, *J* = 1.4 Hz, 3H), 1.28-1.13 (m, 6H), 0.92 (d, *J* = 24.6 Hz, 9H), 0.85-0.68 (m, 6H). m/z: 523.4 [M+H]⁺

### [Example 4]

### Cell Viability Assay

FaDu or A549 cell lines were seeded into 96-well plates at a density of 3,000 cells per well and incubated at 37 °C, 5% CO₂. After 24 hours, 100 µL of drug at 9 concentrations (from 1000 nM, serially diluted 1/5) was added to the cell line. As the drugs, the compound of Chemical Formula 2 prepared according to Example 1, SC209 (MedChemExpress), and MMAE were treated, and at this time, a control group (drug concentration 0) without drug treatment was also prepared. After incubation under incubator conditions (37 °C, 5% CO₂) for 3 days, 100 µL of CellTiter-Glo reagent (using CellTiter-Glo^{®} Luminescent Cell Viability Assay kit (Promega, G7571)) was added to each well of the plate, followed by pipetting. After incubation for 10 minutes (RT), luminescence was measured, and when the luminescence value at drug concentration 0 is regarded as 100%, the concentration showing 50% luminescence value is the IC₅₀ value.

**[Table 1]**

| Compound | A549 IC₅₀(nM) | FaDu IC₅₀(nM) |
|---|---|---|
| Compound of Chemical Formula 2 | 22.59 | 6.168 |
| SC209 | 47.99 | 12.71 |
| MMAE | 3.717 | 1.574 |

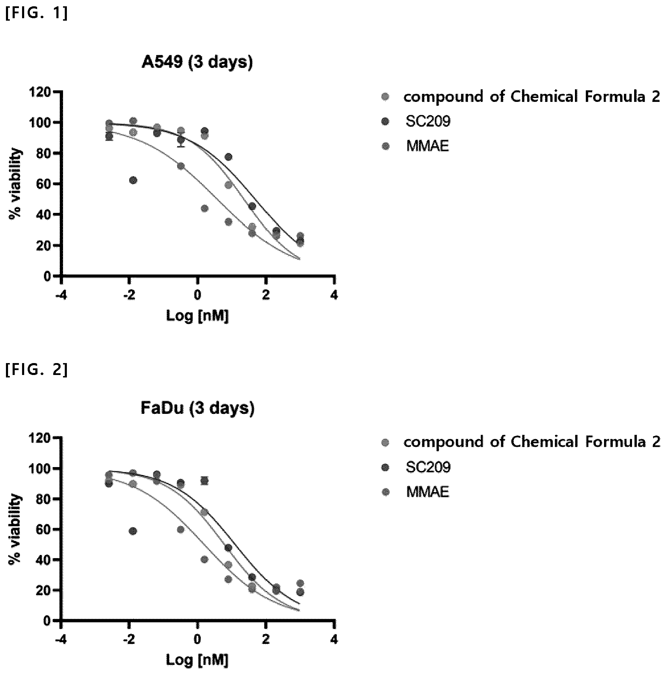

The above results are shown in FIG. 1, FIG. 2 and Table 1. Referring to the results, it can be confirmed that when the compound of the present invention was treated on the lung cancer cell line (A529) and the head and neck squamous cell carcinoma cell line (FaDu), the cell viability was decreased to a level similar to that observed with SC209 and MMAE treatment. That is, the compound of the present invention (in this Example, Chemical Formula 2) has a cytotoxic effect against cancer cells. As confirmed in the above Example, the compound according to the present invention exhibits a cytotoxic effect against cancer cells, and therefore, the compound may be used as a composition for preventing or treating cancer comprising the compound.

Although not particularly limited in present specification, in addition to a composition comprising the compound alone, it may also be provided in a form of a pharmaceutical formulation conventionally used in the art. For example, the compound of the present invention may be provided in the form of a drug-linker in which the compound is conjugated as a drug with a linker, a drug-carrier in which the compound is conjugated as a drug with a carrier, or a drug-linker-carrier form (wherein the carrier includes an antibody, an aptamer, a repebody, and the like). More specifically, a drug in the form of an antibody-drug conjugate comprising the compound as a payload may be provided, and this may be variously used in compositions for combination therapy and the like. It will be apparent to those skilled in the art that all of the drug-linker, drug-carrier, and drug-linker-carrier forms will have a cytotoxic effect equivalent to or similar to the cytotoxic activity possessed by the compound of the present invention alone, and that conjugates in which the compound of the present invention is bound to known peptide-based linkers such as GGFG and val-cit, or acid-sensitive linkers such as CL2A, are also encompassed within the scope of the present invention.

The present invention has been described with reference to preferred embodiments. However, it will be understood by those skilled in the art that the present invention can be implemented in various modified forms without departing from the essential characteristics of the invention. Accordingly, the disclosed embodiments are to be considered as illustrative, not limiting. The scope of the present invention is defined by the claims, not by the foregoing description, and all differences falling within the equivalent scope thereof shall be interpreted as being included within the present invention.

## Claims

1. A compound of Chemical Formula 1, a derivative thereof, an isomer thereof, a pharmaceutically acceptable salt thereof or a solvate thereof: 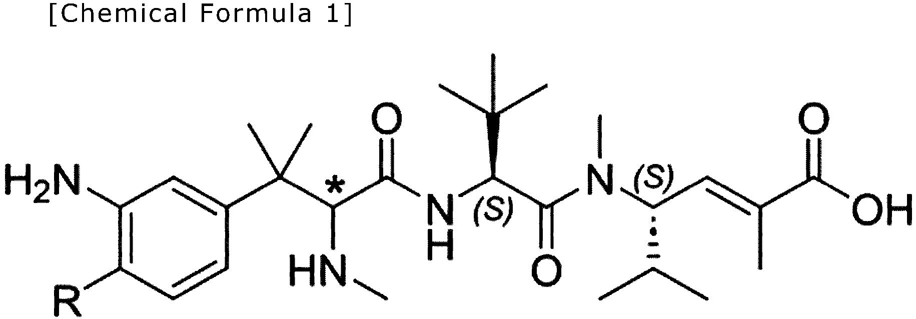 wherein R, in Chemical Formula 1, is selected from the group consisting of halogen, hydroxy, carboxyl group, C1-C6 alkyl and C1-C6 alkoxy.

2. The compound of claim 1, a derivative thereof, an isomer thereof, a pharmaceutically acceptable salt thereof or a solvate thereof,
wherein the derivative of Chemical Formula 1 is a compound represented by following Chemical Formula 2 or 3:

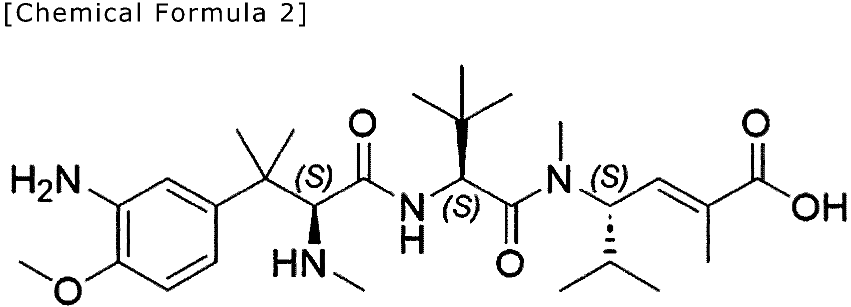

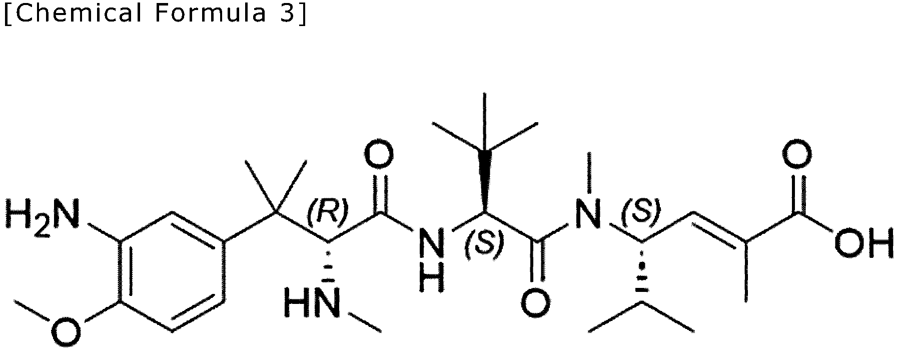

3. The compound of claim 1, a derivative thereof, an isomer thereof, a pharmaceutically acceptable salt thereof or a solvate thereof,
wherein the derivative of Chemical Formula 1 is a compound represented by following Chemical Formula 4 or 5:

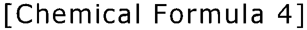

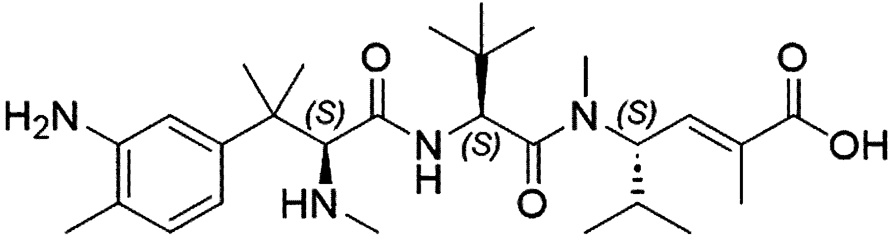

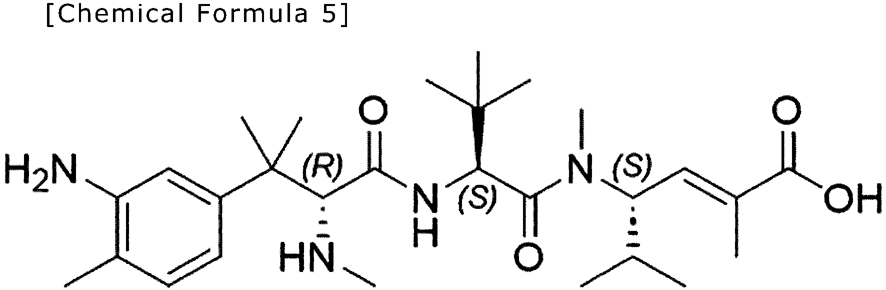

4. The compound of claim 1, a derivative thereof, an isomer thereof, a pharmaceutically acceptable salt thereof or a solvate thereof,
wherein the derivative of Chemical Formula 1 is a compound represented by following Chemical Formula 6 or 7:

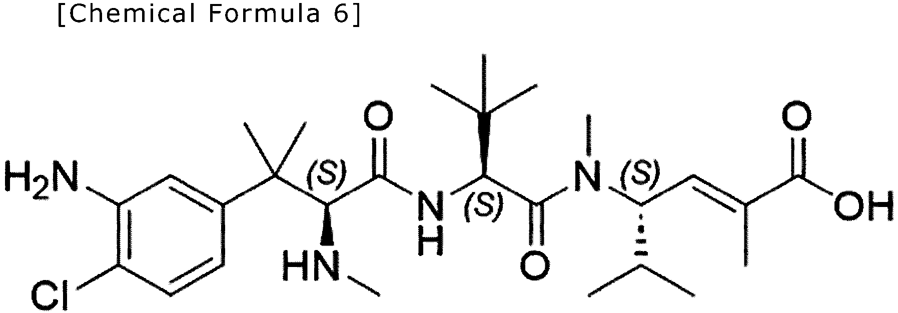

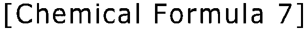

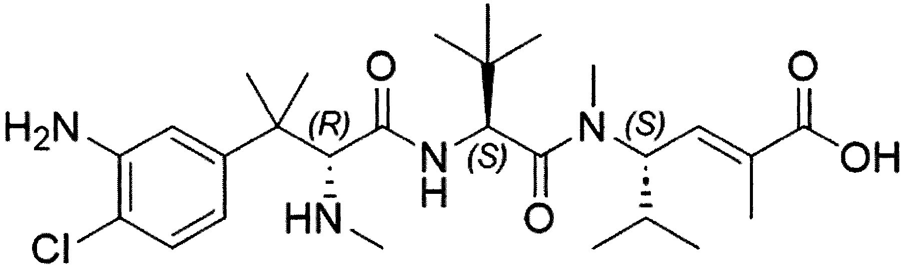

5. A carrier-drug conjugate, comprising a compound of Chemical Formula 1, a derivative thereof, an isomer thereof, a pharmaceutically acceptable salt thereof or a solvate thereof; and a carrier conjugated to the compound: 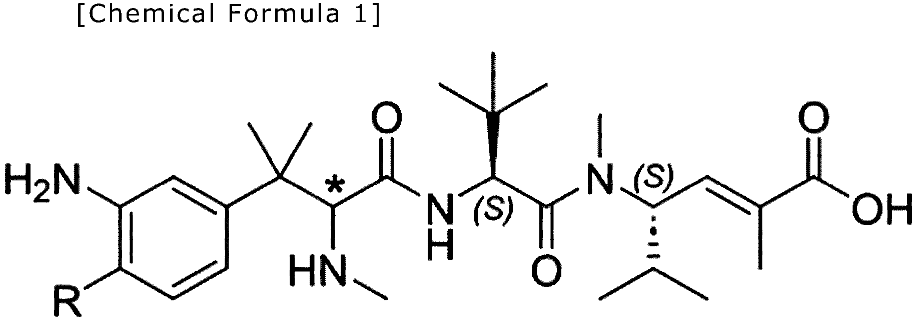 wherein R, in Chemical Formula 1, is selected from the group consisting of halogen, hydroxy, carboxyl group, C1-C6 alkyl and C1-C6 alkoxy.

6. The carrier-drug conjugate of claim 5,
wherein the derivative of Chemical Formula 1 is a compound represented by following Chemical Formula 2 or 3:

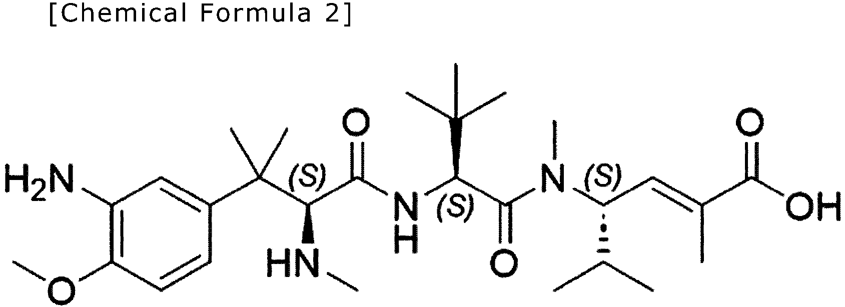

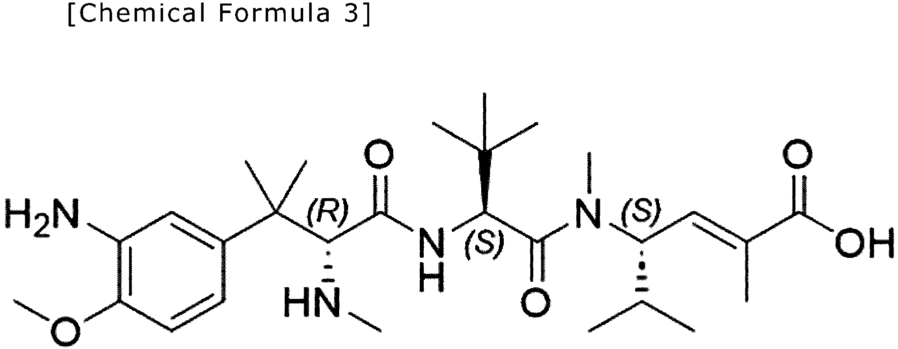

7. The carrier-drug conjugate of claim 5,
wherein the derivative of Chemical Formula 1 is a compound represented by following Chemical Formula 4 or 5:

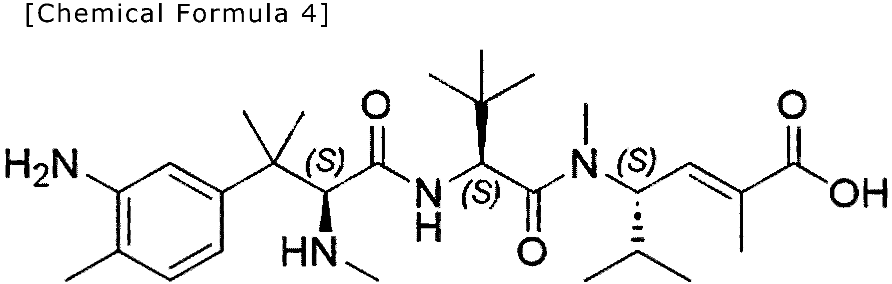

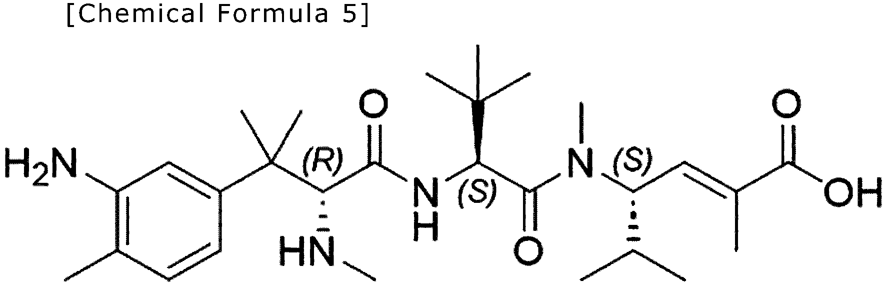

8. The carrier-drug conjugate of claim 5,
wherein the derivative of Chemical Formula 1 is a compound represented by following Chemical Formula 6 or 7:

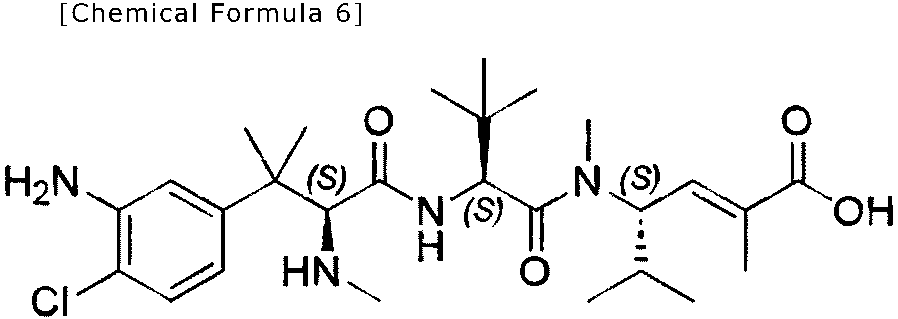

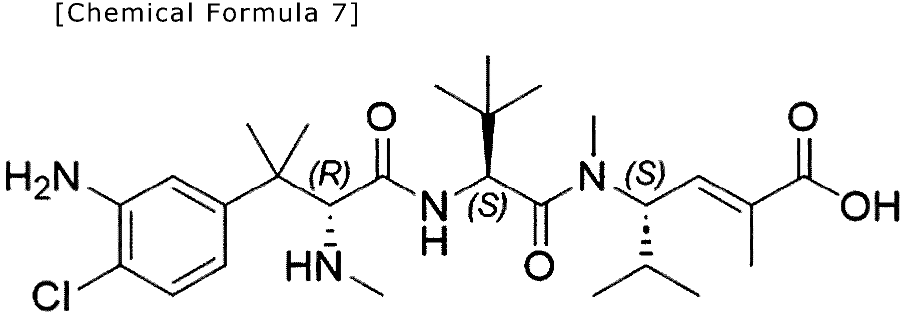

9. The carrier-drug conjugate of claim 5,
wherein the carrier-drug conjugate is in a form in which the carrier is conjugated to the drug via a linker.

10. The carrier-drug conjugate of claim 5,
wherein the linker comprises GGFG or val-cit.

11. The carrier-drug conjugate of claim 8,
wherein the carrier is an antibody, a peptide, a repebody or and aptamer.

12. The carrier-drug conjugate of claim 8,
Wherein the antibody is at least one selected from the group consisting of Urelumab, Utomilumab, Bebtelovimab, Aducanumab, Bapinezumab, Crenezumab, Donanemab, Gantenerumab, Lecanemab, Solanezumab, Nesvacumab, Evinacumab, Enoblituzumab, Omburtamab, Belimumab, Ianalumab, Tabalumab, Bertilimumab, Mogamulizumab, Leronlimab, Siplizumab, Foralumab, Muromonab-CD3, Otelixizumab, Teplizumab, Ibalizumab, Tregalizumab, Zanolimumab, Itolizumab, Efalizumab, Inebilizumab, Tafasitamab, Tositumomab, Ocrelizumab, Ofatumumab, Rituximab, Ublituximab, Veltuzumab, Epratuzumab, Basiliximab, Daclizumab, Varlilumab, Lulizumab, Iratumumab, Lintuzumab, Daratumumab, Felzartamab, Isatuximab, Mezagitamab, Bleselumab, Dacetuzumab, Iscalimab, Lucatumumab, Mitazalimab, Sotigalimab, Dapirolizumab, Apamistamab, Ligufalimab, Magrolimab, Alemtuzumab, Crizanlizumab, Inclacumab, Cusatuzumab, Oleclumab, Milatuzumab, Galiximab, Carotuximab, Adecatumumab, Eptinezumab, Erenumab, Fremanezumab, Galcanezumab, Zolbetuximab, Onartuzumab, Eculizumab, Pozelimab, Ravulizumab, Lacnotuzumab, Axatilimab, Cabiralizumab, Emactuzumab, Ipilimumab, Quavonlimab, Tremelimumab, Zalifrelimab, Cetuximab, Depatuxizumab, Futuximab, Imgatuzumab, Matuzumab, Modotuximab, Necitumumab, Nimotuzumab, Panitumumab, Tomuzotuximab, Zalutumumab, Batoclimab, Nipocalimab, Rozanolixizumab, Burosumab, Farletuzumab, Dinutuximab, Naxitamab, Ragifilimab, Gimsilumab, Lenzilumab, Mavrilimumab, Namilumab, Otilimab, Plonmarlimab, Codrituzumab, Margetuximab, Pertuzumab, Trastuzumab, Datopotamab, Patritumab, Seribantumab, Duligotuzumab, Ficlatuzumab, Rilotumumab, Alomfilimab, Anifrolumab, Emapalumab, Ligelizumab, Omalizumab, Cixutumumab, Dalotuzumab, Figitumumab, Ganitumab, Teprotumumab, Bermekimab, Canakinumab, Gevokizumab, Briakinumab, Ustekinumab, Anrukinzumab, Cendakimab, Lebrikizumab, Tralokinumab, Brodalumab, Bimekizumab, Ixekizumab, Secukinumab, Brazikumab, Guselkumab, Mirikizumab, Risankizumab, Tildrakizumab, Nemolizumab, Imsidolimab, Spesolimab, Pascolizumab, Dupilumab, Depemokimab, Mepolizumab, Reslizumab, Benralizumab, Clazakizumab, Olokizumab, Siltuximab, Sirukumab, Ziltivekimab, Levilimab, Sarilumab, Satralizumab, Tocilizumab, Abituzumab, Favezelimab, Fianlimab, Ieramilimab, Relatlimab, Simtuzumab, Abagovomab, Oregovomab, Tanezumab, Ivuxolimab, Rocatinlimab, Tavolimab, Telazorlimab, Vonlerolizumab, Alirocumab, Bococizumab, Ebronucimab, Evolocumab, Frovocimab, Ongericimab, Tafolecimab, Dostarlimab, Balstilimab, Camrelizumab, Cemiplimab, Geptanolimab, Nivolumab, Pembrolizumab, Penpulimab, Pidilizumab, Prolgolimab, Retifanlimab, Sasanlimab, Serplulimab, Sintilimab, Spartalizumab, Tislelizumab, Toripalimab, Ezabenlimab, Zimberelimab, Atezolizumab, Avelumab, Cosibelimab, Sugemalimab, Durvalumab, Envafolimab, Suvratoxumab, Denosumab, Zilovertamab, Elotuzumab, Domvanalimab, Etigilimab, Ociperlimab, Tiragolumab, Vibostolimab, Surzebiclimab, Cobolimab, Sabatolimab, Concizumab, Marstacimab, Adalimumab, Golimumab, Infliximab, Certolizumab, Conatumumab, Tigatuzumab, Tezepelumab, Gatipotuzumab, Cabiralizumab, Bevacizumab, Brolucizumab, Ranibizumab, Olinvacimab, Icrucumab, Ramucirumab, Caplacizumab, Abrilumab, Etrolizumab, Vedolizumab, Intetumumab, Natalizumab, Obrindatamab, Elranatamab, Linvoseltamab, Teclistamab, Epcoritamab, Glofitamab, Mosunetuzumab, Odronextamab, Flotetuzumab, Vibecotamab, Catumaxomab, Cibisatamab, Talquetamab, Ubamatamab, Emfizatamab, Blinatumomab, Amivantamab, Emicizumab, Zenocutuzumab, Zanidatamab, Tibulizumab, Naptumomab, Belantamab, Pivekimab, Praluzatamab, Coltuximab, Denintuzumab, Loncastuximab, Ibritumomab, Inotuzumab, Epratuzumab, Moxetumomab, Brentuximab, Gemtuzumab, Vadastuximab, Lorvotuzumab, Polatuzumab, Tusamitamab, Telisotuzumab, Rovalpituzumab, Depatuxizumab, Farletuzumab, Mirvetuximab, Disitamab, Anetumab, Enfortumab, Sacituzumab, Vobarilizumab, Cadonilimab, Vudalimab, Tebotelimab, Ivonescimab, Erfonrilimab, Ozoralizumab, Faricimab, Vanucizumab, and Navicixizumab.

13. A drug-linker, comprising a compound of Chemical Formula 1, a derivative thereof, an isomer thereof, a pharmaceutically acceptable salt thereof or a solvate thereof; and a carrier conjugated to the compound: 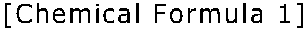 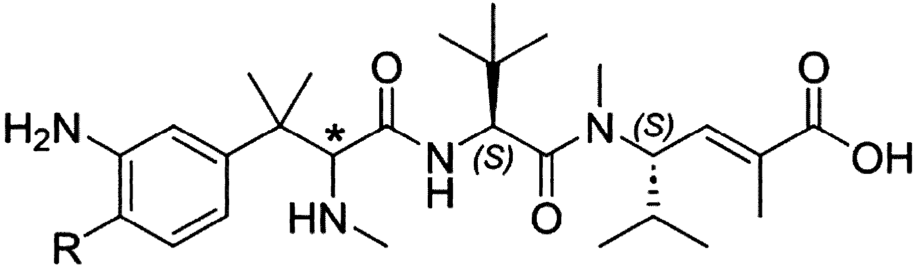 wherein R, in Chemical Formula 1, is selected from the group consisting of halogen, hydroxy, carboxyl group, C1-C6 alkyl and C1-C6 alkoxy.

14. The drug-linker of claim 13,
wherein the linker comprises GGFG or val-cit.

15. A pharmaceutical composition for preventing or treating cancer, comprising a compound of Chemical Formula 1, a derivative thereof, an isomer thereof, a pharmaceutically acceptable salt thereof or a solvate thereof: 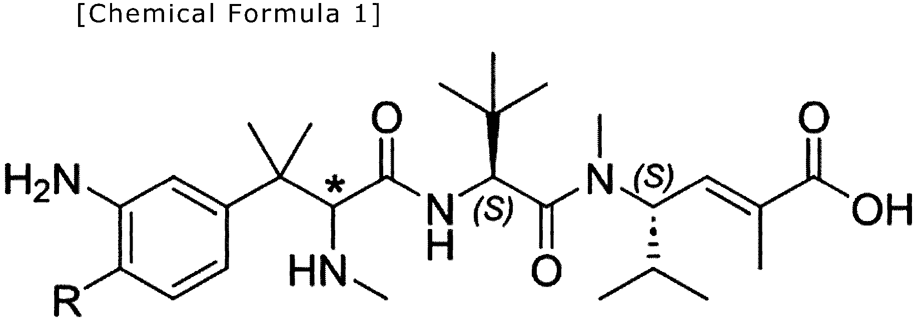 wherein R, in Chemical Formula 1, is selected from the group consisting of halogen, hydroxy, carboxyl group, C1-C6 alkyl and C1-C6 alkoxy.

16. The pharmaceutical composition of claim 15,
wherein the compound of Chemical Formula 1 is conjugated to carrier via a linker.

17. The pharmaceutical composition of claim 15,
wherein the cancer is at least one selected from the group consisting of pseudomyxoma, intrahepatic cholangiocarcinoma, hepatoblastoma, liver cancer, thyroid cancer, colon cancer, testicular cancer, myelodysplastic syndrome, glioblastoma, oral cancer, lip cancer, mycosis fungiodes, acute myeloid leukemia, acute lymphoblastic leukemia, basal cell carcinoma, ovarian epithelial carcinoma, ovarian germ cell carcinoma, male breast cancer, brain cancer, pituitary adenoma, multiple myeloma, gallbladder cancer, bile duct cancer, colorectal cancer, chronic myelogenous leukemia, chronic lymphocytic leukemia, retinoblastoma, choroidal melanoma, ampulla of Vater cancer, bladder cancer, peritoneal cancer, parathyroid cancer, adrenal cancer, paranasal sinus cancer, non-small cell lung cancer, tongue cancer, astrocytoma, small cell lung cancer, pediatric brain cancer, pediatric lymphoma, childhood leukemia, small bowel cancer, meningioma, esophageal cancer, glioma, renal pelvis cancer, kidney cancer, heart cancer, duodenal cancer, malignant soft tissue cancer, malignant bone cancer, malignant lymphoma, malignant mesothelioma, malignant melanoma, eye cancer, vulvar cancer, ureteral cancer, urethral cancer, unknown primary neoplasm, gastric lymphoma, gastric cancer, gastric carcinoma, gastrointestinal stromal cancer, Wilms' cancer, breast cancer, Triple-negative breast cancer(TNBC), sarcoma, penile cancer, pharyngeal cancer, gestational trophoblastic disease, cervical cancer, endometrial cancer, uterine sarcoma, prostate cancer, metastatic bone cancer, metastatic brain cancer, mediastinal cancer, rectal cancer, occipital carcinoid tumors, vaginal cancer, spinal cancer, vestibular schwannoma, pancreatic cancer, salivary gland cancer, Kaposi's sarcoma, Paget's disease, tonsil cancer, squamous cell carcinoma, lung adenocarcinoma, lung cancer, squamous cell lung cancer, skin cancer, anal cancer, rhabdomyosarcoma, laryngeal cancer, pleural cancer, blood cancer, and thymic carcinoma.
